# EUROPEAN PATENT SPECIFICATION

(11) **EP 0 698 097 B1**
(45) Date of publication and mention of the grant of the patent: **16.08.2001**
(21) Application number: 94916201.0
(22) Date of filing: 28.04.1994
(51) Int. Cl.: C12N 15/13, C07K 14/435, C12P 21/08, A61K 39/395

(54) **PRODUCTION OF ANTIBODIES OR (FUNCTIONALIZED) FRAGMENTS THEREOF DERIVED FROM HEAVY CHAIN IMMUNOGLOBULINS OF CAMELIDAE**
HERSTELLUNG VON ANTIKÖRPERN ODER FUNKTIONSTÜCHTIG GEMACHTEN TEILEN DAVON, ABGELEITET VON SCHWEREN KETTEN VON IMMUNGLOBULINEN VON CAMELIDAE
PRODUCTION D'ANTICORPS OU DE FRAGMENTS FONCTIONNALISES D'ANTICORPS, DERIVES DES IMMUNOGLOBULINES A CHAINE LOURDE DE CAMELIDAE

(30) Priority: 29.04.1993 EP 93201239; 19.05.1993 EP 93201454; 15.07.1993 EP 93202079
(43) Date of publication of application: 28.02.1996
(73) Proprietor: UNILEVER N.V., 3013 AL Rotterdam (NL); UNILEVER PLC, London EC4P 4BQ (GB); VRIJE UNIVERSITEIT BRUSSEL, 1050 Brussel (BE)
(72) Inventor: HAMERS, Raymond, B-1640 Sint-Genesius-Rode (BE); HAMERS-CASTERMAN, Cécile, B-1640 Sint-Genesius-Rode (BE); MUYLDERMANS, Serge, Victor, M., B-1560 Hoeilaart (BE); FRENKEN, Leon, Gerardus, J., NL-3011 MP Rotterdam (NL); VERRIPS, Cornelis, Theodorus, NL-3142 KB Maassluis (NL)
(74) Representative: Dekker, Enno E.J.
(86) International application number: EP9401442
(87) International publication number: WO9425591

(56) References cited:
- EP-A- 0 256 421
- EP-A- 0 584 421
- WO-A-94/04678
- NATURE vol. 363, no. 6428 , 3 June 1993 , LONDON, GB pages 446 - 448 C. HAMERS-CASTERMAN ET AL. 'Naturally occurring antibodies devoid of light chains.' cited in the application
- FEBS LETTERS vol. 339, no. 3 , 21 February 1994 , AMSTERDAM, THE NETHERLANDS pages 285 - 290 J. DAVIES ET AL. ''Camelising' human antibody fragments: NMR studies on VH domains.'

## Description

The present invention relates to a process for the production of antibodies or (functionalized) fragments thereof derived from heavy chain immunoglobulins of *Camelidae* and is partly based on research investigations carried out at the Free University of Brussels. A **draft publication** thereon already submitted to the periodical Nature and communicated to the present applicants by Prof. R. Hamers reads as follows.

### FUNCTIONAL HEAVY CHAIN IMMUNOGLOBULINS IN THE CAMELIDS

Random association of V_{L} and V_{H} repertoires contributes considerably to antibody diversity (1). The diversity and the affinity are then increased by hypermutation in B-cells located in germinal centres (2). Except in the heavy chain disease (3), naturally occurring heavy chain antibodies have not been described, although antigen binding has been demonstrated for separated heavy chains (4) or cloned V_{H} domains (5). The presence of considerable amounts IgG like material of 100 Kd in the serum of the camel (*Camelus dromedarius*) (6) was confirmed. These molecules are composed of heavy chain dimers and are devoid of light chains. Nevertheless they bear an extensive antigen binding repertoire, a finding which questions the role of the light chains in the camel. Camel heavy chain IgGs lack the C_{H}1, which in one IgG class might be structurally replaced by an extended hinge. Heavy chain IgGs are a feature of all camelids. These findings open perspectives in engineering of antibodies.

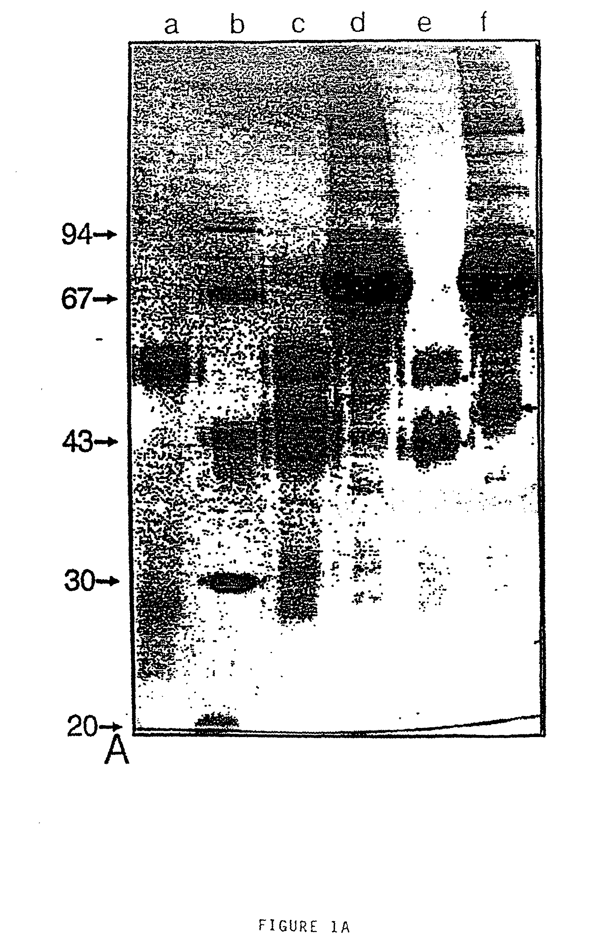

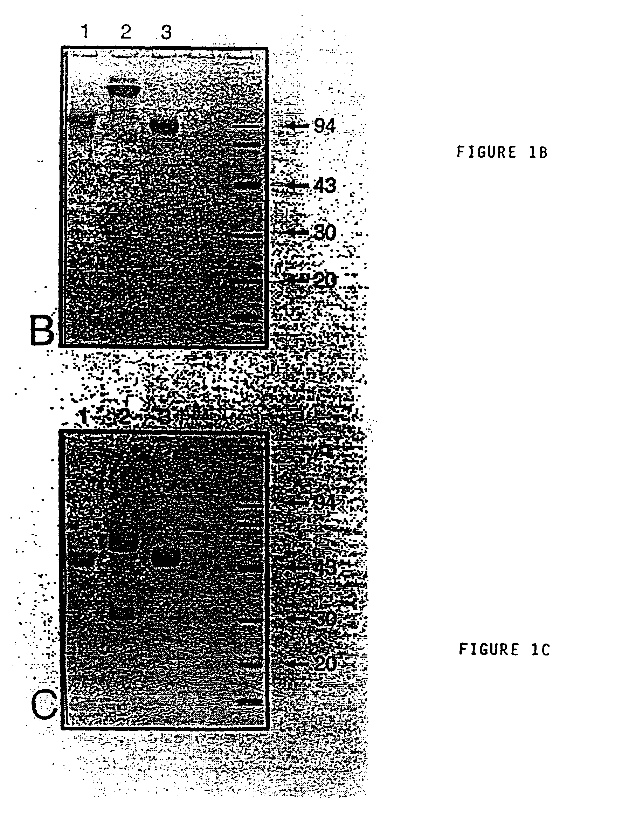

By a combination of affinity chromatography on Protein A and Protein G, three quantitatively important fractions corresponding to subclasses of IgG can be isolated from the serum of camels (*Camelus dromedarius*) (Fig. 1A, lanes c-f).
One fraction (IgG₁) contains molecules of 170 Kd (Fig. 1B, lane 2) which upon reduction yield 50 Kd heavy chains and large 30 kD light chains (Fig. 1C, lane 2). The two other immunoglobulin fractions contain molecules of approximately 100 Kd (Fig. 1B, lanes 1 and 3) which upon reduction yield only heavy chains of respectively 46 Kd (IgG₂ fraction hinding only to Protein A) (Fig. 1C, lane 3) and 43 Kd (IgG₃ fraction binding to Protein A and Protein G) (Fig. 1C, lane 1). These two IgG classes appear to lack the light chain completely.

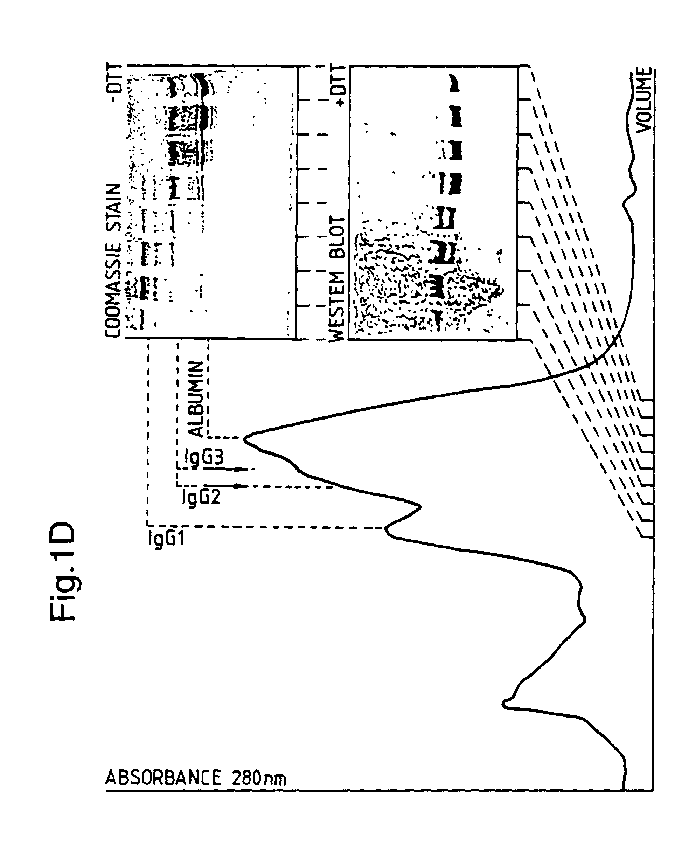

To exclude the possibility that the light chains were only weakly associated with the heavy chains and lost during the selective purification, whole serum was size fractionated by gel filtration. Coomassie blue staining of unreduced fractions revealed the sequential elution of the 170 Kd IgG₁ followed by the incompletely resolved isotypes IgG₂ and IgG₃ (90 Kd) (Fig. 1D, upper inset). Immunostaining of the same fractions after reduction confirmed that the light chains were present solely in the 50 Kd heavy chain containing fractions (Fig. 1D, lower inset).

A comparative study of old world camelids (*Camelus bactrianus* and *Camelus dromedarius*) and new world camelids (*Lama pacos*, *Lama glama* and *Lama vicugna*) showed that heavy chain immunoglobulins are abundant in the sera of all species examined (data not shown) and total up to 75% of the molecules binding to protein A.

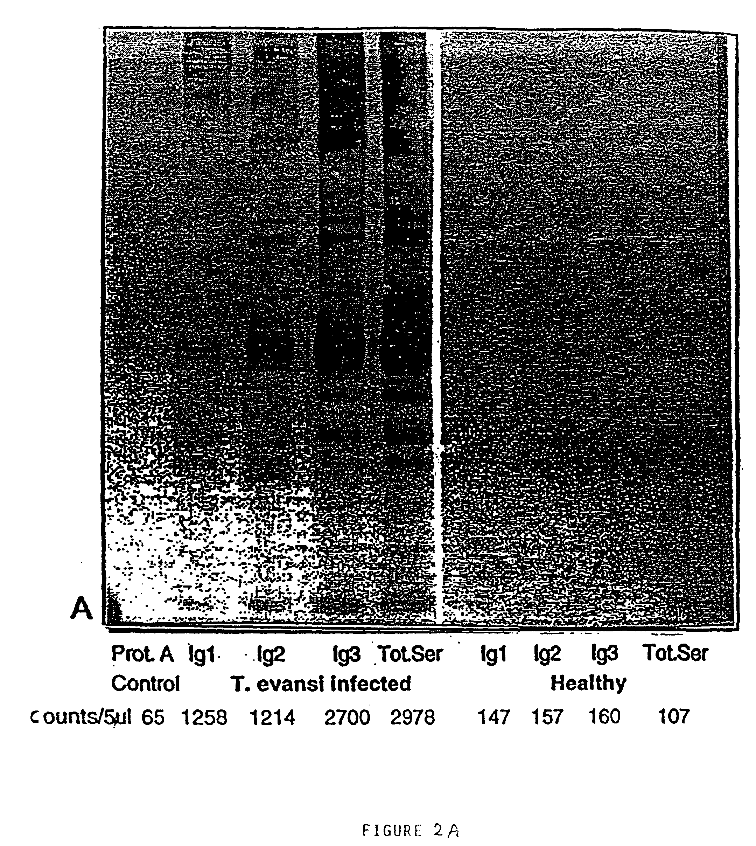

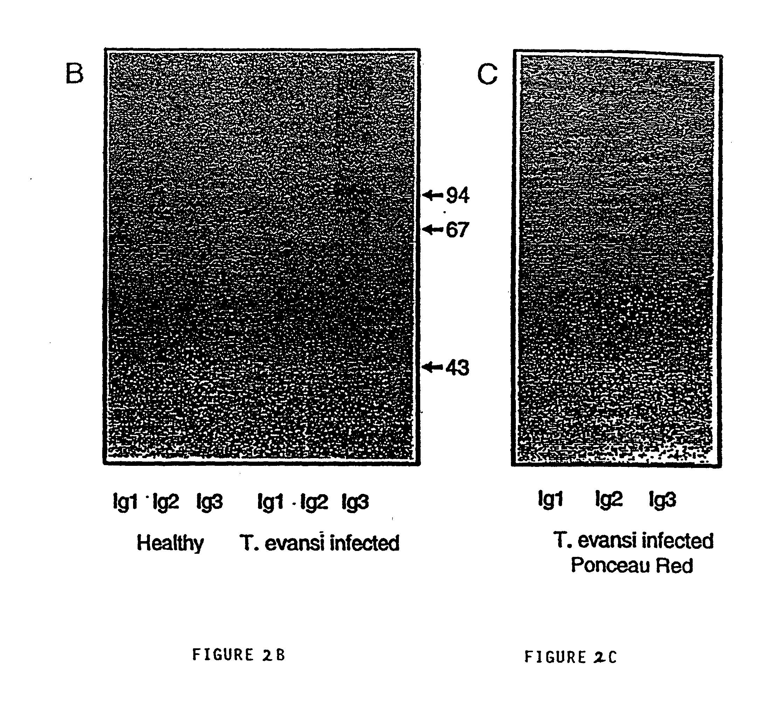

The abundance of the heavy chain immunoglobulins in the serum of camelids raises the question as to whether they bear an extensive antigen binding repertoire. This question could be answered by examining the IgG₁, IgG₂ and IgG₃ fractions from the serum of camels (*Camelus dromedarius*) with a high antitrypanosome titer (7). In radio-immunoprecipitation, purified fractions of IgG₁, IgG₂ and IgG₃ derived from infected camels were shown to bind a large number of antigens present in a ³⁵*S* methionine labelled trypanosome lysate (Fig. 2A), indicating an extensive repertoire complexity for the three IgG classes. Conversely, in blotting experiments, ³⁵*S* methionine labelled trypanosome lysate binds to SDS-PAGE separated IgG₁, IgG₂ and IgG₃ obtained from infected animals (Fig. 2B). These findings indicate that the heavy chains alone can generate an extensive repertoire and question the obligatory contribution of the light chain to the useful antibody repertoire in the camelids.

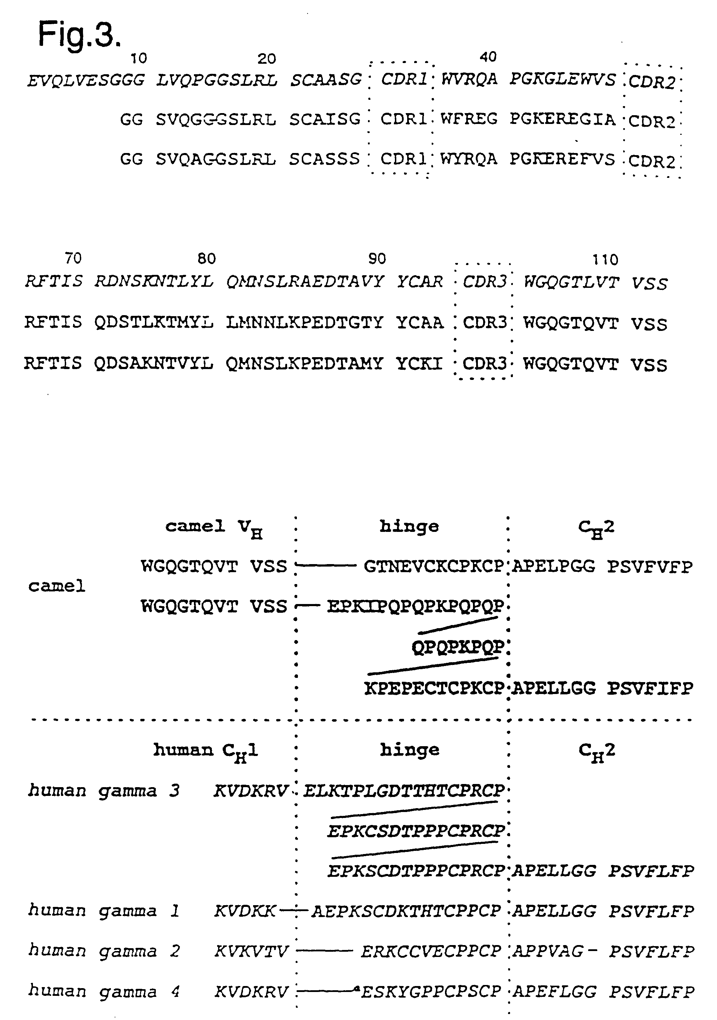

The camelid γ2 and γ3 chains are considerably shorter than the normal mammalian γ or camel γ1 chains. This would suggest that, as in the case of heavy chain disease (3), deletions have occurred in the C_{H}1 protein domain (8,9). To address this question, cDNA was synthesized from camel spleen mRNA and the sequences between the 5' end of the V_{H} and the C_{H}2 were amplified by a Polymerase Chain Reaction (PCR), and cloned. Seventeen clones presenting a different V_{H} sequence were isolated and sequenced. Their most striking feature was the complete lack of the C_{H}1 domain, the last framework (FR4) residues of the V_{H} region being immediately followed by the hinge (Fig. 3, lower part). The absence of the C_{H}1 domain clarifies two important dilemmas.

First, immunoglobulin heavy chains are normally not secreted unless the heavy chain chaperoning protein or BIP (10) has been replaced by the L chain (11), or alternatively the C_{H}1 domain has been deleted (3,8,9). Secondly, isolated heavy chains from mammalian immunoglobulins tend to aggregate, but are only solubilized by light chains (8,12) which bind to the C_{H}1 and the V_{H} domains (13).

14 of the 17 clones were characterized by a short hinge sequence with a length equal to that of human IgG₂ and IgG₄ (14) (Fig. 3). The other 3 had a long hinge sequence containing the 'EPK' hinge motif found in human IgG₁ and IgG₃ (14).
They possess the C_{H}2 'APELL/P' motif also found in human IgG₁ and IgG₃ (see SEQ. ID. NO: 1-2), and which is associated with mammary transport of bovine IgG₁ (15). On basis of molecular weight, we expect the "short hinge" clones to correspond to IgG₃ and the "long hinge" clones to IgG₂.

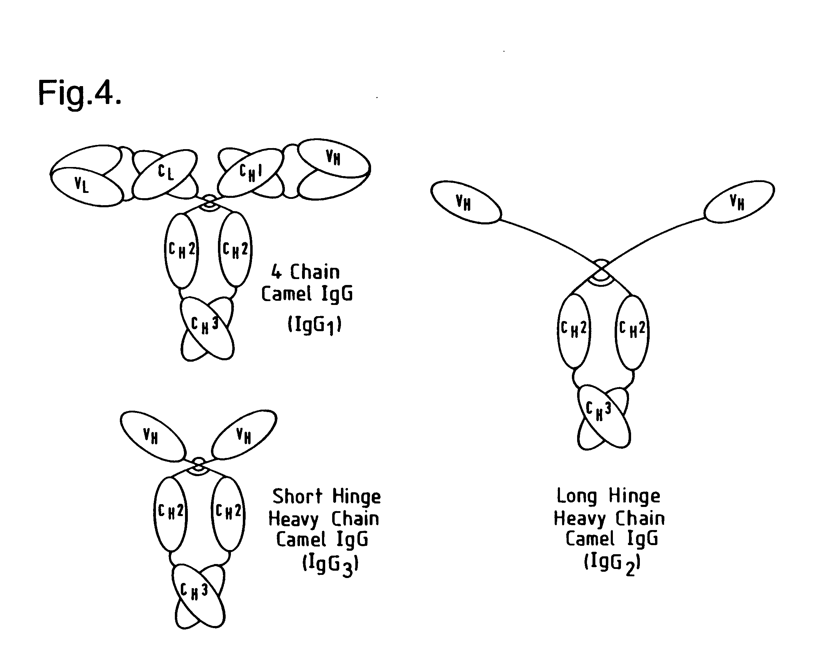

In the short hinge containing antibody, the extreme distance between the extremities of the V_{H} regions will he of the order of 80 Å corresponding to twice the size of a single domain of 40 Å (2xV_{H}) (16). This could be a severe limitation for agglutinating, cross linking or complement fixation (17,18). In the long hinge containing immunoglobulin the absence of C_{H}1 might be compensated by the extremely long hinge itself, composed of a 12 fold repeat of the sequence Pro-X (X=Gln, Glu, Lys) (Fig. 3 & 4). NMR (19) and molecular modelling (20) of Pro-X repeats present in the TonB protein of *E*. *coli* (X=Glu, Lys) and the membrane procyclin of trypanosomes (X=Asp, Glu) indicate that these repeated sequences function as rigid rodlike spacers with a diameter of 8 Å and a rise of 2.9 Å per residue. Assuming the same geometry, the long hinge would be 70 Å which compensates for the absence of the C_{H}1 domain.

The binding site of heavy chain antibodies cannot form the pocket resulting from adjoining light and heavy chain V regions and the residues of the V_{H} which normally interact with V_{L} will he exposed to solvent (3,5,13). It was found that leucine at position 45 conserved in 98% of human and murine V_{H} sequences (14), and crucial in the V_{H}-V_{L} association (13), can be replaced by an arginine (Fig. 3, upper part). This substitution is in accordance with both the lost contact with a V_{L} domain and an increased solubility.

Unlike myeloma heavy chains which result mainly from C_{H}1 deletion in a single antibody producing cell (21) the camelid heavy chain antibodies have emerged in a normal immunological environment and it is expected that they will have undergone the selective refinement in specificity and affinity accompanying B cell maturation (1, 2). The obtention of camelid heavy chain antibodies could therefore be an invaluable asset in the development and engineering of soluble V_{H} domains (5) or of new immunologicals for diagnostic, therapeutic or biochemical purposes.

### REFERENCES

1. Tonegawa, *S*. *Nature* **302,** 575-581 (1983).
2. Jacob, J., Kelsoe, G., Rajewski, K., & Weiss, U. *Nature* **354,** 389-392 (1991).
3. Fleischman J.B., Pain R.H. & Porter R.R. *Arch*.*Biochem*.*Biophys* **Suppl. 1,** 174-180 (1962).
4. Utsumi, S. & Karush, F. *Biochemistry 3,* 1329-1338 (1964).
5. Ward, E.S., Güssow, D., Griffiths, A.d., Jones, P.T. & Winter G. *Nature* **341,** 544-546 (1989).
6. Ungar-Waron H., Eliase E., Gluckman A. and Trainin Z. *Isr*. *J*. *Vet. Med.* **43,** 198-203 (1987).
7. Bajyana Songa, E., & Hamers R. *Ann*.*Soc*.*Belge Méd*.*Trop*. **68,** 233-240 (1988).
8. Seligmann M., Mihaesco E., Preud'homme J.-L., Danon F. & Brouet J.-C. *Immun*.*Rev*. **48,** 145-167 (1979).
9. Traunecker, A., Schneider, J., Kiefer, H., Karjalaien, K., *Nature* **339,** 68-70 (1989).
10. Henderschot L.M., Bole D., Köhler, G. & Kearney, J.F. *J*. *Cell Biol.* **104,** 761-767 (1987).
11. Henderschot L.M. *J*.*Cell Biol*. **111,** 829-837 (1990).
12. Roholt O., Onoue K. & Pressman D. *Proc*.*Natn*.*Acad*.*Sci*. *USA* **51,** 173-178 (1964).
13. Chothia, C., Novotny, J., Bruccoleri, R., Karplus, M. *J*. *Mol*.*Biol*. **186,** 651-663 (1985).
14. Kabat E.A., Wu, T.T., Reid-Miller, M., Perry H.M. & Gottesman, K.S. *Sequences of Proteins of Immunological Interest* 511 (U.S. Dept of Health and Human Services, US Public Health Service, National Institutes of Health, Bethesda, 1987).
15. Jackson, T., Morris, B.A, Sanders, P.G. *Molec*.*Immun*. **29,** 667-676 (1992).
16. Poljak RJ. *et al*. *Proc*.*Natn*.*Acad*.*Sci*. *USA* **70,** 3305-3310 (1973).
17. Dangl J.L., *et al*. *EMBO J.* **7,** 1989-1994 (1988).
18. Schneider W.P. *et al*. *Proc*.*Natn*.*Acad*.*Sci USA* **85,** 2509-2513 (1988).
19. Evans, J.S. *et al*. *FEBS Lett.* **208,** 211-216 (1986).
20. Roditi, I. *et al*. *J*.*Cell Biol.* 108, 737-746 (1989).
21. Dunnick, W., Rabbits, T.H., Milstein, C. *Nucl*.*Acids Res.,* **8,** 1475-1484 (1980).
22. Bülow, R., Nonnengässer, C., Overath, P. *Mol*.*Biochem*.*Parasitol*. **32,** 85-92 (1989).
23. Sambrook, J., Fritsch, E.F. & Maniatis, T. *Molecular Cloning: A Laboratory Manual* 2nd Edn (Cold Spring Harbor Laboratory Press, New York, 1989).
24. Sastry, L *et al*. *Proc*.*Natn*.*Acad*.*Sci*. *USA* **86,** 5728-5732 (1989).
25. Sanger, F., Nicklen, S. & Coulson, A.R. *Proc*.*Natn*.*Acad*.*Sci*. *USA* **74,** 5463-5467 (1977).
26. Klein, J. *Immunology* (Blackwell Scientific Publications, London, 1990).

### Figure 1 Characterisation and purification of camel IgG classes on Protein A, Protein G and gel filtration.

(A) The fraction of *C*. *dromedarius* serum adsorbed on Protein A shows upon reduction on SDS-PAGE three heavy chain components of respectively 50, 46, and 43 Kd (bands between dots), absent in the non adsorbed fraction (lane d), and light chain components of around 30 Kd (lane c) considerably larger than rabbit light chain (lane a, rabbit IgG). The fractions adsorbed on Protein G (lane e) lack the 46 Kd heavy chain which remains in the non adsorbed fraction (lane f). Lane b contains a size marker.
(B and C) By differential adsorption and elution on Protein G and Protein A, the IgG fractions containing 43 Kd (lane 1), 46 Kd (lane 3) and 50 Kd (lanes 2) heavy chains were purified and analysed on SDS-PAGE in absence (B) or presence (C) of DTT.
(D) Whole camel serum (0.1 ml) was fractionated by gel filtration on a Superdex 200 column using 150 mM NaCl, 50 mM sodium phosphate buffer pH 7.0 as eluent. Affinity purified IgG₂ and IgG₃ elute at the positions indicated by arrows. The fractions of interest were further analysed by SDS-PAGE with or without prior reduction. The protein contents as visualized by Coomassie blue (without reduction, upper inset) are compared with the immunoglobulins from the same fractions (after reduction with DTT, lower inset) as revealed by Western blotting with a rabbit anti-camel-IgG (lower inset).

METHODS. 5 ml of *C*. *dromedarius* serum is adsorbed onto a 5 ml Protein G Sepharose (Pharmacia) column, and washed with 20 mM phosphate buffer, pH 7.0. Upon elution with 0.15 M NaCl, 0.58 % acetic acid (pH 3.5), IgG₃ of 100 Kd is eluted which upon reduction yields heavy chains of 43 Kd (lane 1, B and C). IgG₁ of 170 Kd can subsequently he eluted with pH 2.7 buffer (0.1 M Gly-HCl). This fraction, upon reduction, yields a 50 Kd heavy chain and a broad light chain band (lane 2, C). The fraction not adsorbed on Protein G is brought on a 5 ml Protein A Sepharose column. After washing and elution with 0,15 M NaCl, 0.58% acetic acid (pH 4.5) IgG₂ of 100 Kd is obtained which consists solely of 46 Kd heavy chains (lane 3, C).

### Figure 2 Repertoire complexity and antigen binding capacity of camel IgG₁, IgG₂ and IgG₃ analysed by radioimmunoprecipitation (A) or Western blotting (B & C).

(A) Serum or purified IgG fractions from healthy or *Trypanoma evansi* infected *C*. *dromedarius* (CATT titer 1/160 (7)) were incubated with labelled trypanosome lysate, recovered with Protein A Sepharose and analysed by SDS-PAGE. The relative counts recovered are inscribed below each lane. No trypanosome proteins hind to the Protein A or to the healthy camel immunoglobulins.
(B) 20 µg of IgG₁, IgG₂ and IgG₃ from healthy and trypanosome infected animals were separated by SDS-PAGE without prior reduction or heating. The electroblotted proteins were incubated with the labelled trypanosome lysate. The IgG₂ shows a single antigen binding component corresponding to the heavy chain immunoglobulin whereas the IgG₃ fraction appears to contain in addition two larger antigen binding components barely detectable by Ponceau Red staining (C). These are possibly Ig classes copurified as immunocomplexes present in the serum of the infected animals.

METHODS. (³⁵S)-methionine labelled *Trypanosoma evansi* lysate (500,000 counts) (22) was incubated (4°C, 1 hour) with 10 µl of serum or, 20 µg of IgG₁, IgG₂ or IgG₃ in 200 µl of 0.4 M NaCl, 10 mM EDTA, 10 mM Tris (pH 8.3), containing 0.1 M TLCK. 10 mg of Protein A SeDharose suspended in 200 µl of the same buffer was added (4°C, 1 hour). After washing and centrifugation, each pellet was resuspended in 75 µl SDS PAGE sample solution containing DTT, and heated for 3 min. at 100°C. After centrifugation, 5 µl of the supernatant was saved for radioactivity counting and the remainder analysed by SDS PAGE and fluorography.
The nitrocellullose filter of the Western blot of purified fractions IgG₁, IgG₂ and IgG₃ was stained with Ponceau Red (C) or incubated with 1% ovalbumin in TST buffer (Tris 10 mM, NaCl 150 mM, Tween 0,05%) (B). The membrane was extensively washed with TST buffer and incubated for 2 hours with (³⁵S)-labelled trypanosome antigen. To avoid unspecific hinding, the labelled trypanosome antigen lysate was filtered (45 µ) and incubated with healthy camel immunoglobulin and ovalbumin adsorbed on a nitrocellulose membrane.

### Figure 3 Amino acid sequences of the V_{H} framework, and hinge/C_{H}2 of Camelus dromedarius heavy chain immunoglobulins, compared to human (italic) V_{H} framework (subgroup III) and hinges of human IgG (14).

METHODS. Total RNA was isolated from a dromedary spleen (23). mRNA was purified with oligo T-paramagnetic heads (PolyATract-Promega). 1 µg mRNA was used for preparing double-strand cDNA (23) after an oligo-dT priming using enzymes provided by Boehringer Mannheim. 5 µg of cDNA was amplified by PCR in a 100 µl reaction mixture (10mM Tris-HCl pH 8.3, 50 mM KC1,15 mM MgCl₂, 0.01% (w/v) gelatine, 200 µM of each dNTP). 25 pmoles of each oligonucleotide of the mouse V_{H} (24), containing a Xhol site, and 5'-CGCCATCAAGGTACCAGT-TGA-3' (see SEQ. ID. NO: 3) were used as primers. The 3' end primer was deduced from partial sequences corresponding to γ chain amino acid 296 to 288 (T.Atarhouch, C. Hamers-Casterman, G. Robinson, private communication) in which one mismatch was introduced to create a KDnI restriction site. After a round of denaturing annealing (94°C for 5 min. and 54°C for 5 min.), 2 U of Taq DNA polymerase were added, to the reaction mixture before subjecting it to 35 cycles of amplification (5). The PCR products were purified by phenol-chloroform extraction followed by HPLC (Genpak-fax column, Waters) and finally by MERMAID (BIO 101, Inc.). After these purification steps, the amplified cDNA was digested with XhoI and KpnI, and ligated into pBluescript.
The clones were sequenced by the dideoxy chain termination method (25). The sequences were translated into amino acids which allowed their assignment to well defined domains of the Ig molecule (14); see SEQ. ID. NO: 4-12

### Figure 4 Schematic representation of the structural organisation of the camel immunoglobulins (adapted from 26).

On the basis of size consideration, the IgG₁ fraction possess probably the normal antibody assembly of two light and two heavy chains. IgG₃ would have a hinge comparable in size to the human IgG₁, IgG₂ and IgG₄. The two antigen binding sites are much closer to each other as this camel IgG lacks the C_{H}1 domain. In the camel IgG₂ the long hinge, being formed of Pro-X repeats (X= Glu, Gln or Lys), most likely adopt a rigid structure (19,20). This long hinge could therefore substitute the C_{H}1 domain and bring the two antigen binding sites of IgG₂ to normal positions.

### --- End of Draft publication ---

### Background of the invention

Already at a very early stage during evolution antibodies have been developed to protect the host organisms against invading molecules or organisms. Most likely one of the earliest forms of antibodies must have been developed in *Agnatha*. In these primitive fishes antibodies of the IgM type consisting of heavy and lights chains have been detected. Also in many other forms of life ranging from amphibians to mammals antibodies are characterized by the feature that they consist of two heavy and two light chains, although the heavy chains of the various classes of immunoglobulins are quite different. These heavy and light chains interact with each other by a number of different physical forces, but interactions between hydrophobic patches present on both the heavy and light chain are always important. The interaction between heavy and light chains exposes the complementarity determining regions (CDRs) of both chains in such a way that the immunoglobulin can bind the antigen optimally. Although individual heavy or light chains have also the capability to bind antigens (Ward *et al.,* Nature **341** (1989) 544-546 = ref. 5 of the above given draft publication) this binding is in general much less strong than that of combined heavy and light chains.
Heavy and light chains are composed of constant and variable domains. In the organisms producing immunoglobulins in their natural state the constant domains are very important for a number of functions, but for many applications of antibodies in industrial processes and products their variable domains are sufficient. Consequently many methods have been described to produce antibody fragments.

One of these methods is characterized by cleavage of the antibodies with proteolytic enzymes like papain and pepsin resulting in (a) antibody fragment comprising a light chain bound via an S-S bridge to part of a corresponding heavy chain formed by proteolytic cleavage of the heavy chain (Fab), or (b) a larger fragment of the antibody comprising two of these Fabs still connected to each other via an S-S bridge in enlargements of the heavy chain parts, indicated with F(ab)₂, respectively (see patent applications EP-A-0125023 (GENENTECH / Cabilly *et al*., 1984) and WO-A-93/02198 (TECH. RES. CENT. FINLAND / Teeri *et al*., 1993) for definitions of these abbreviations). The disadvantage of the enzymatic route is that the production of whole antibodies is expensive and the enzymatic processing increases the costs of these fragments even more. The high costs of antibody fragments block the application of these fragments in processes and products outside the pharmaceutical industry.

Another method is based on linkage on DNA level of the genes encoding (parts of) the heavy chain and the light chain. This linkage and the subsequent production of these chimeric immunoglobulins in microorganisms have been described (for Fab fragments see e.g, Better *et al*., Science **240** (1988) 1041-1043, for Fᵥ fragments (combination of variable fragments of the heavy chain (V_{H}) and light chain (V_{L}) still connected to each other by non-covalent binding interactions) see e.g. Skerra *et al*., Science **240** (1988) 1938, and for single chain Fᵥ fragments (ScFᵥ; an Fᵥ fragment in which the two variable fragments are linked to each other by a linker peptide) see e.g. Bird *et al*., Science **242** (1988) 423-426. Provided that an appropriate signal sequence has been placed in front of the single chain V_{H} and V_{L} antibody fragment (ScFᵥ), these products are translocated in *E*. *coli* into the periplasmic space and can be isolated and activated using quite elaborate and costly procedures. Moreover the application of antibody fragments produced by *E*. *coli* in consumer products requires extensive purification processes to remove pyrogenic factors originating from *E*. *coli.* For this and other reasons the production of ScFᵥ in microorganisms that are normally used in the fermentation industry, like prokaryotes as *Streptomyces* or *Bacillus* (see e.g. Wu *et al*. Bio/Technology **11** (1993) 71) or yeasts belonging to the genera *Saccharomyces* (Teeri *et al*., 1993, *supra*), *Kluyveromyces*, *Hansenula*, or *Pichia* or moulds belonging to the genera *Aspergillus* or *Trichoderma* is preferred. However with a very few exceptions the production of ScFᵥ antibodies using these systems proved to be impossible or quite poor. Although the exact reasons for the poor production are not well known, the use of linkers between the V_{H} and V_{L} chains not designed for secretion (Teeri *et al*., 1993, *supra)* may be a reason.

Another reason may he incorrect folding of ScFᵥ. The frameworks and to a limited extend the CDRs of variable domains of light and heavy chains interact with each other. It has been described by Chothia *et al.* (J. Mol. Biol. **186** (1985) 651-663 = ref. 13 of the above given draft publication) that this interaction involves amino acids at the following positions of the variable region of the heavy chain: 35, 37, 39, 44-45, 47, 100-103 and 105 (numbering according to Kabat *et al*., In *"Sequences of Proteins of Immunological Interest,* Public Health Service, NIH, Washington DC, 1983 = ref. 14 of the above given draft publication). Especially leucine at position 45 is strongly conserved and the whole apolar side chain of this amino acid seems to be involved in the interaction with the light chain. These strong interactions may fold the ScFᵥ into a structure that can not be translocated in certain types of lower eukaryotes.

Thus the use of a linker in the production of ScFᵥ for connecting a V_{H} chain to a V_{L} chain, might negatively influence either the translocation, or the folding of such ScFᵥ or both.

Not prior-published European patent application 92402326.0 filed 21.08.92 (C. Casterman & R. Hamers) discloses the isolation of new animal-derived immunoglobulins devoid of light chains (also indicated as heavy chain immunoglobulins), which can especially originate from animals of the camelid family (*Camelidae*). This European patent specification, now publicly available as EP-A1-0 584 421, is incorporated herein by reference. These heavy chain immunoglobulins are characterized in that they comprise two heavy polypeptide chains sufficient for the formation of one or more complete antigen binding sites, whereby a complete antigen binding site means a site which will alone allow the recognition and complete binding of an antigen, which can he verified by any known method regarding the testing of the binding affinity. The European patent specification further discloses methods for isolating these heavy chain immunoglobulins from the serum of *Camelidae* and details of the chemical structure of these heavy chain immunoglobulins. It also indicates that these heavy chain immunoglobulins and derivatives thereof can be made by using recombinant DNA technology in both prokaryotes and eukaryotes. The present invention relates to a further development of the work disclosed in that prior-filed but not prior-published European specification.

Due to the absence of light chains in most of the immunoglobulins of *Camelidae* such linkers are not necessary, thereby avoiding the above-mentioned potential problems.
As described above in the draft publication for Nature, now publicly available as Nature 363 (3 June 1993) 446-448, and in the not prior-published European patent application 92402326.0 (*supra*) it was surprisingly found that the majority of the protein A-binding immunoglobulins of *Camelidae* consists just of two heavy chains and that these heavy chains are quite different from common forms of heavy chains, as the C_{H}1 domain is replaced by a long or short hinge (indicated for IgG₂ and IgG₃, respectively, in Figure 4 of the above given draft publication for Nature).
Moreover these heavy chains have a number of other features that make them remarkably different from the heavy chains of common immunoglobulins.
One of the most significant features is that they contain quite different amino acid residues at those positions involved in binding to the light chain, which amino acids are highly conserved in common immunoglobulins consisting of two heavy and two light chains (see Table 1 and SEQ. ID. NO: 13-31).

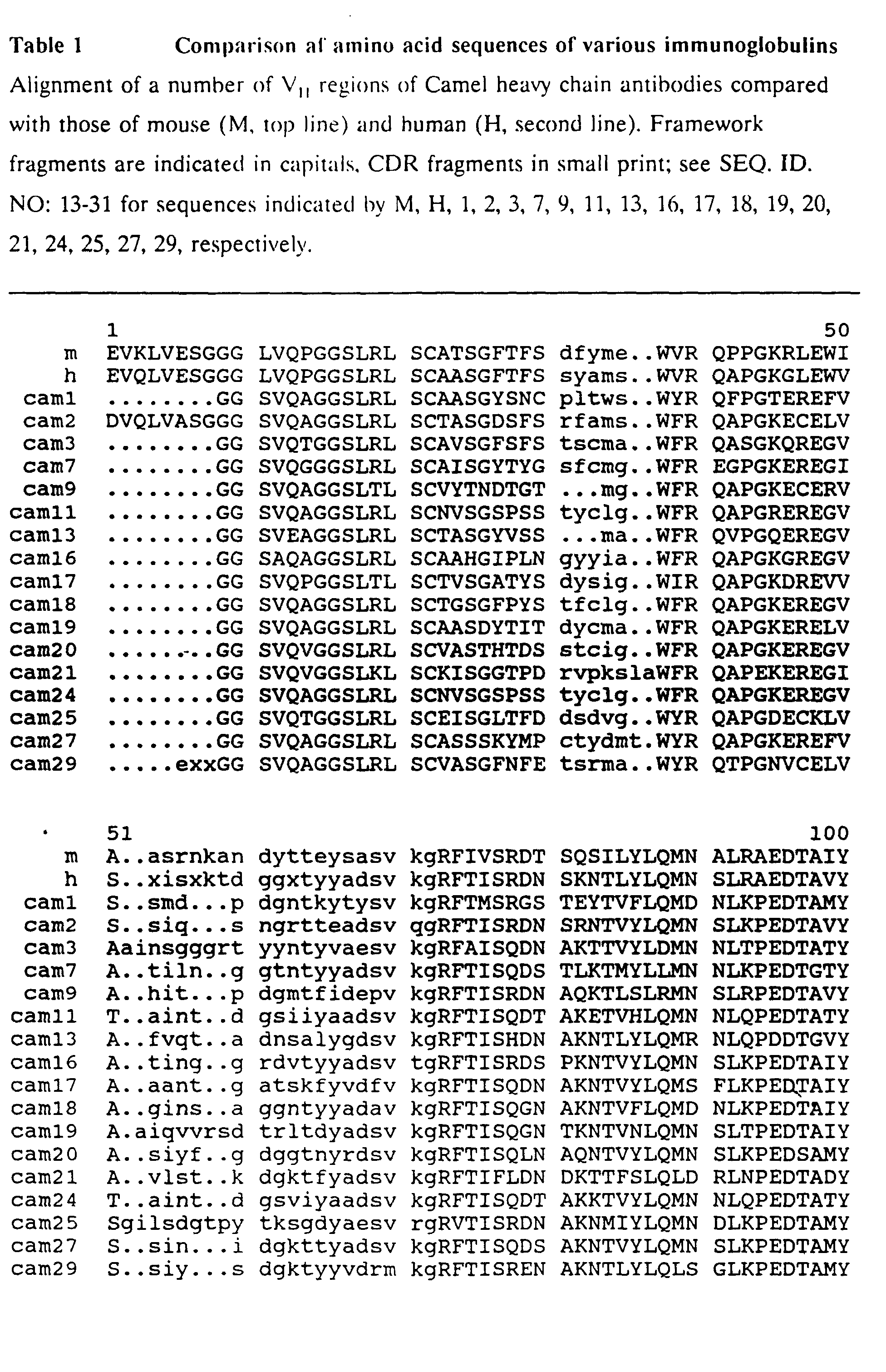

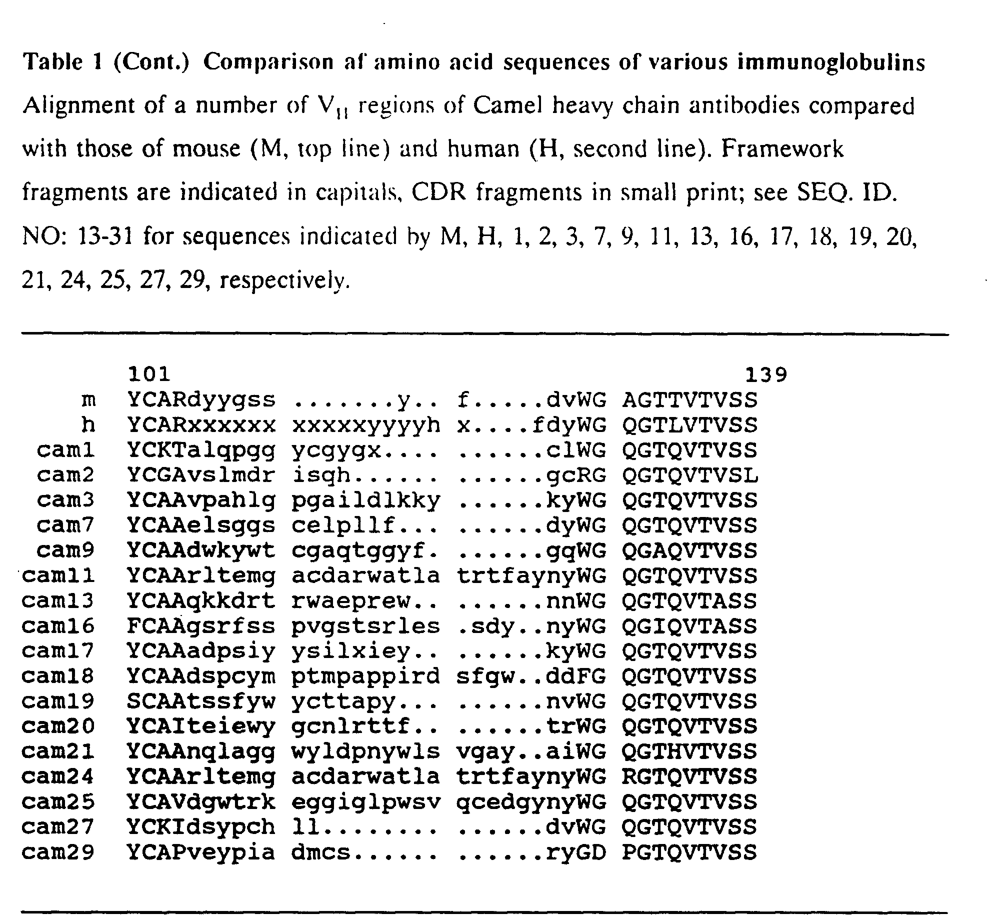

For example, according to Pessi *et al.* (1993) a subdomain portion of a V_{H} region of common antibodies (containing both heavy chains and light chains) is sufficient to direct its folding, provided that a cognate V_{L} moiety is present. Thus it might be expected from literature on the common antibodies that without V_{L} chains proper folding of heavy chains cannot he achieved. A striking difference between the common antibodies and the *Camelidae*-derived heavy chain antibodies is, that the highly conserved apolar amino acid leucine (L) at place 45 present in common. antibodies is replaced in most of the *Camelidae*-derived heavy chain antibodies by the charged amino acid arginine (R), thereby preventing binding of the variable region of the heavy chain to that of the light chains.
Another remarkable feature is that one of the CDRs of the heavy chains of this type of immunoglobulins from *Camelidae*, CDR3, is often much longer than the corresponding CDR3 of common heavy chains. Besides the two conserved cysteines forming a disulphide bridge in common V_{H} fragments, the *Camelidae* V_{H} fragments often contain two additional cysteine residues, one of which often is present in CDR3.
According to the present inventors these features indicate that CDR3 may play an important role in the binding of antigens by these heavy chain antibodies and can compensate for the absence of light chains (also containing CDRs) in binding of antigens by immunoglohulins in *Camelidae*.
Thus, as the heavy chains of *Camelidae* do not have special features for interacting with corresponding light chains (which are absent), these heavy chains are very different from common heavy chains of immunoglobulins and seem intrinsically more suitable for secretion by prokaryotic and lower eukaryotic cells.

The present inventors realized that these features make both intact heavy chain immunoglobulins of *Camelidae* and fragments thereof very attractive for their production by microorganisms. The same holds for derivatives thereof including functionalized fragments. In this specification the term "functionalized fragment" is used for indicating an antibody or fragment thereof to which one or more functional groups, including enzymes and other binding polypeptides, are attached resulting in fusion products of such antibody fragment with another biofunctional molecule.

### Summary of the invention

In a broad sense the invention provides a process for the production of an antibody or a fragment or functionalized fragment thereof using a transformed lower eukaryotic host containing an expressible DNA sequence encoding the antibody or (functionalized) fragment thereof, wherein the antibody or (functionalized) fragment thereof is derived from a heavy chain immunoglobulin of *Camelidae* and is devoid of light chains, and wherein the lower eukaryotic host is a mould or a yeast. Thus the lower eukaryotic host can he a mould, e.g. belonging to the genera *Aspergillus* or *Trichoderma*, or a yeast belonging to the yeast genera *Saccharomyces*, *Kluyveromcyes*, *Hansenula*, or *Pichia*. Preferably the fragments still contain the whole variable domain of these heavy chains.
The invention also provides methods to produce such heavy chain immunoglobulins or (functionalized) fragments thereof in which methods the framework or the CDRs of these heavy chains are modified by random or directed mutagenesis in such a way that the mutated heavy chain is optimized for secretion by the host microorganism into the fermentation medium.
Another embodiment of the invention is that CDRs can he grafted on these optimized frameworks (compare grafting of CDRs on human immunoglobulins as described by e.g. Jones *et al*., Nature **321** (1986) 522). These CDRs can be obtained from common antibodies or they may originate from heavy chain immunoglobulins of *Camelidae*. The binding properties may be optimized by random or directed mutagenesis. Thus in a process according to the invention an antibody or (functionalized) fragment thereof derived from a heavy chain immunoglobulin of *Camelidae* can be produced which comprises a CDR different from the CDR belonging to the natural antibody ex *Camelidae* which is grafted on the framework of the variable domain of the heavy chain immunoglobulin ex *Camelidae*.
The invention also provides a method for the microbiological production of catalytic antibodies. These antibodies are preferably raised in *Camelidae* against transition state molecules following procedures similar to the one described by Lerner *et al*., Science **252** (1991) 659-667. Using random or site-directed mutagenesis such catalytic antibodies or fragments thereof can be modified in such a way that the catalytic activity of these (functionalized) antibodies or fragments can be further improved.
For preparing modified heavy chain antibodies a process according to the invention is provided, in which the DNA sequence encodes a modified heavy chain immunoglobulin or a (functionalized) fragment thereof derived from *Camelidae* and being devoid of light chains, and is made by random or directed mutagenesis or both. Thus the resulting immunoglobulin or (functionalized) fragment thereof is modified such that
- it is optimized for secretion by the lower eukaryotic host into the fermentation medium, or
- its hinding properties (kₒₙ and k_{off}) are optimized, or
- it has acquired a metal chelating activity, or

Another particular embodiment of the present invention relates to genes encoding fusion proteins consisting of both a heavy chain immunoglobulin from *Camelidae* or part thereof and a second protein or another polypeptide, e.g. an enzyme, in particular an oxido-reductase, and to expression products of such genes. By means of the heavy chain immunoglobulin (fragment) the protein or enzyme can be guided to a target thereby increasing the local efficiency of the protein or enzyme significantly.
Thus according to this embodiment of the invention a process is provided, in which the functionalized antibody or fragment thereof comprises a fusion protein of both a heavy chain immunoglobulin from *Camelidae* or a fragment thereof and another polypeptide, e.g. an enzyme, preferably an oxido-reductase.

As a result of a process according to the invention known products may be produced, e.g. antibodies also produced by *Camelidae*, but many of the possible products will be new products, thus the invention also provides new products obtainable by a process according to the invention.
The products so produced can be used in compositions for various applications.

### Brief Description of the Figures

Figures 1-4 were already described above in the draft publication.
- Figure 1: Characterisation and purification of camel IgG classes on Protein A, Protein G and gel filtration.
- Figure 2: Repertoire complexity and antigen binding capacity of camel IgG₁, IgG₂ and IgG₃ analysed by radioimmunoprecipitation (A) or Western blotting (B & C).
- Figure 3: Amino acid sequences of the V_{H} framework, and hinge/C_{H}2 of *Camelus dromedarius* heavy chain immunoglobulins, compared to human (italic) V_{H} framework (subgroup III) and hinges of human IgG (14); see SEQ. ID. NO: 4-12.
- Figure 4: Schematic representation of the structural organisation of the camel immunoglobulins (adapted from 26).
- Figure 5: DNA and amino acid sequences of the Camel V_{H} fragments followed by the Flag sequence as present in pB03 (Figure 5A), pB09 (Figure 5B) and pB24 (Figure 5C); see SEQ. ID. NO: 32-37.
- Figure 6: Nucleotide sequence of synthetic DNA fragment cloned into pEMBL9 (Example 1); see SEQ. ID. NO: 38-41.
- Figure 7: Schematic drawing of plasmid pUR4423
- Figure 8: Schematic drawing of plasmid pUR4426
- Figure 9: Schematic drawing of plasmid pUR2778
- Figure 10: Schematic drawing of plasmid pUR4429
- Figure 11: Schematic drawing of plasmid pUR4430
- Figure 12: Schematic drawing of plasmid pUR4445

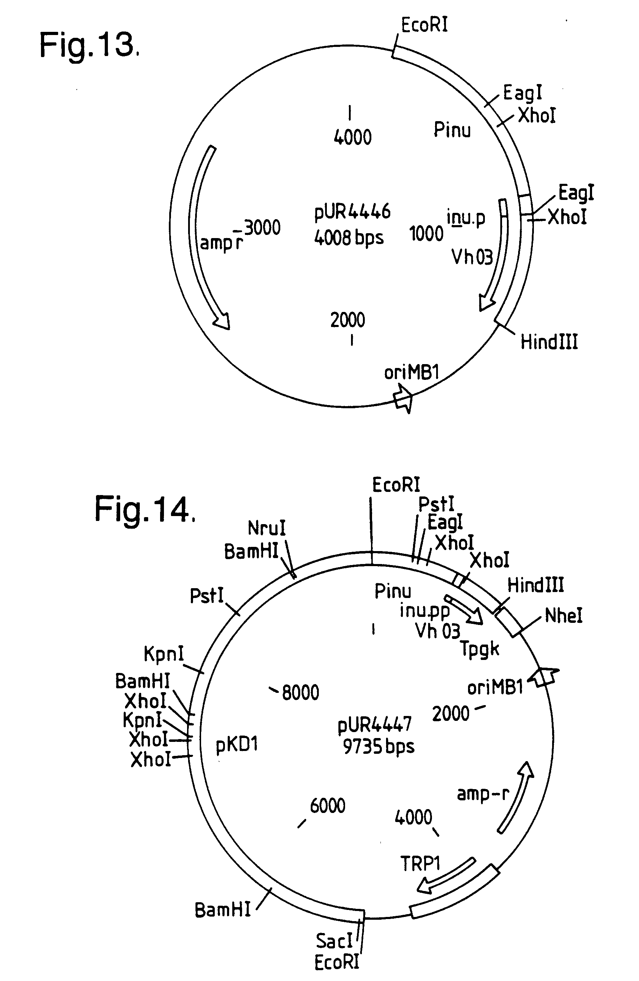

- Figure 13: Schematic drawing of plasmid pUR4446
- Figure 14: Schematic drawing of plasmid pUR4447

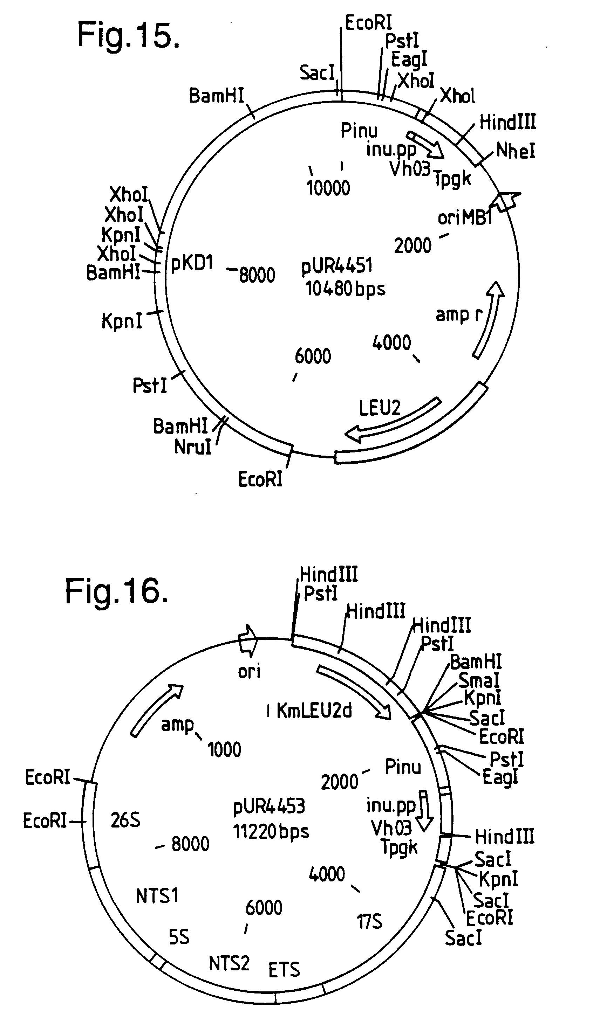

- Figure 15: Schematic drawing of plasmid pUR4451
- Figure 16: Schematic drawing of plasmid pUR4453
- Figure 17: Schematic drawings of plasmids pUR4437 and pUR4438

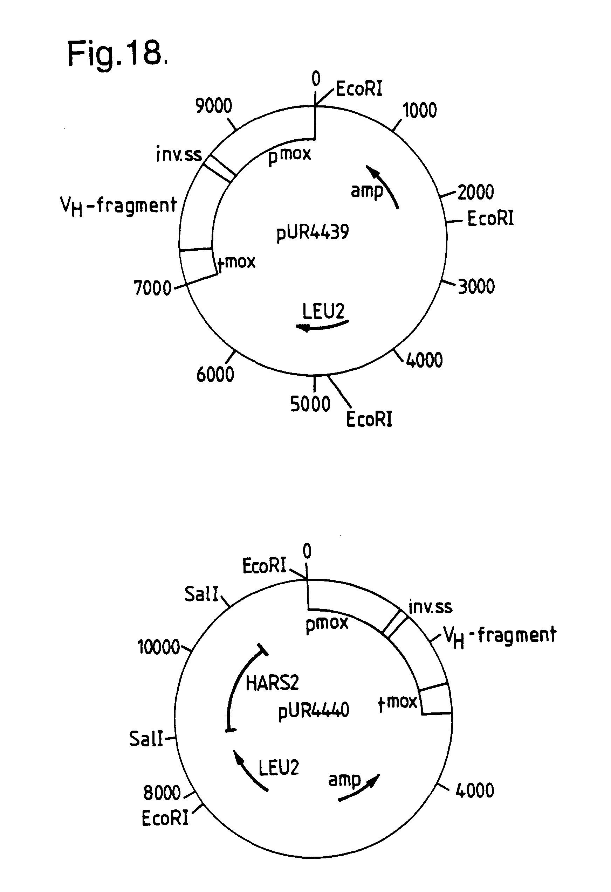

- Figure 18: Schematic drawings of plasmids pUR4439 and pUR4440
- Figure 19: Nucleotide sequence of synthetic DNA fragment cloned into pEMBL9 (Example 6); see SEQ. ID. NO: 42-45.
- Figure 20: Schematic drawing of plasmid pAW14B.
- Figure 21: Western hlot analysis of culture medium of *S*. *cerevisiae* transformants containing pUR4423M (see A) or pUR4425M (see B). Samples were taken after 24 (see 1) or 48 hours (see 2). For pUR4425M two hands were found due to glycosylation of the antibody fragment.

### Detailed description of the invention

The present invention relates to the production of antibodies or (functionalized) fragments thereof derived from heavy chain immunoglobulins of *Camelidae* by eukaryotes, more in particular by lower eukaryotes such as yeasts belonging to the genera Saccharomyces, Kiuyveromyces, Hansenula, or Pichia, and fungi.
Therefore, mRNA encoding immunoglobulins of *Camelidae* was isolated and transcribed into cDNA according to the procedures described in the above given draft publication and not prior-published European patent application 92402326.0. In each case primers for the PCR reaction directed to the N-terminus of the V_{H} domain and PCR primers that either hybridize with the C-terminal regions of the V_{H} domain or with the short or large hinge regions as described in the above given draft publication, or with the C-terminal region of the C_{H}2 or C_{H}3 domains can be used. In this way structural genes can be obtained encoding the following fragments of heavy chain immunoglobulins of *Camelidae* (Table 2).

**Table 2.**

| The various forms of immunoglobulins of *Camelidae* that can be expressed in microorganisms. | |
|---|---|
| a. | the variable domain of a heavy chain; |
| b. | the variable domain and the short hinge of a heavy chain; |
| c. | the variable domain and the long hinge of a heavy chain; |
| d. | the variable domain, the C_{H}2 domain, and either the short or long hinge of a heavy chain; |
| e. | a complete heavy chain, including either the short or long hinge. |

According to procedures described in detail in the Examples these cDNAs can be integrated into expression vectors.
Known expression vectors for *Saccharomyces, Kluyveromcyes, Hansenula, Pichia* and *Aspergillus* can he used for incorporating a cDNA or a recombinant DNA according to the invention. The resulting vectors contain the following sequences that are required for expression: (a) a constitutive, or preferably an inducible, promoter; (b) a leader or signal sequence; (c) one of the structural genes as described in Table 2 and (d) a terminator. If the vector is an episomal vector, it preferably comprises an origin of replication as well as a selection marker, preferably a food grade selection marker, (EP-A-487159, UNILEVER / Leenhouts *et al*.). If the vector is an integration vector, then it preferably comprises sequences that ensure integration and a selection marker in addition to the sequences required for expression of the structural gene encoding a form of the heavy chain immunoglobulin of *Camelidae* or derivatives thereof. The preferred sequences for integration are sequences encoding ribosomal DNA (WO 91/00920, 1991, UNILEVER / Giuseppin *et* al.) whereas the selection marker will he preferably a food grade marker.
For *Saccharomyces* the preferred inducible promoter is the GAL7 promoter (EP-A-0255153, UNILEVER / Fellinger *et al.);* for *Kluyveromyces* the preferred inducible promoter is the inulinase promoter (not yet published EP application 92203932.6, UNILEVER / Toschka & Verbakel, which is incorporated herein by reference); for *Hansenula* or *Pichia* the preferred inducible promoter is the methanol-oxidase promoter (Sierkstra *et al*., Current Genetics **19** (1991) 81-87) and for *Aspergillus* the preferred inducible promoter is the endo-xylanase promoter (not prior-published PCT application PCT/EP 92/02896, UNILEVER / Gouka *et al*., now publicly available as WO-A-93/12237, which is incorporated herein by reference).
To achieve efficient secretion of the heavy chain immunoglobulin or parts thereof the leader (secretion) sequences of the following proteins are preferred: invertase and α-factor for *Saccharomyces*, inulinase for *Kluyveromyces*, invertase for *Hansenula* or *Pichia* (Sierkstra *et al*., 1991 *supra*) and either glucoamylase or xylanase for *Aspergillus* (not prior-published PCT application WO-A-93/12237, *supra).* As food-grade selection markers, genes encoding anabolic functions like the leucine2 and tryptophan3 are preferred (Giuseppin *et al*. 1991, *supra).* The present invention describes the heterologous production of (functionalized) derivatives or fragments of immunoglobulins in a microorganism, which immunoglobulins in nature occur not as a composite of heavy chains and light chains, but only as a composite of heavy chains. Although the secretion mechanism of mammals and microorganisms is quite similar, in details there are differences that are important for developing industrial processes.
To obtain frameworks of the heavy chain immunoglobulins, that are optimally secreted by lower eukaryotes, genes encoding several different heavy chains can be cloned into the coat protein of bacteriophages and subsequently the frameworks of these heavy chain immunoglobulins can he mutated using known PCR technology, e.g. Zhou *et al*., (1991). Subsequently the mutated genes can be been cloned in *Saccharomyces* and *Aspergillus* and the secretion of the mutated genes can be compared with the wild type genes. In this way frameworks optimized for secretion may be selected.
Alternatively these structural genes can he linked to the cell wall anchoring part of cell wall proteins, preferably GPI-linked cell wall proteins of lower eukaryotes, which result in the expression of a chimeric protein on the cell wall of these lower eukaryotes (not prior-published EP application 92202080.5, UNILEVER / Klis *et al*., now publicly available as International (PCT) patent application WO-A-94/01567, which is incorporated herein by reference).
Both methods have the advantage that the binding parts of the immunoglobulins are well exposed to the surrounding of the cell, microorganism, or phage and therefore can bind antigens optimally. By changing the external conditions the binding rates and dissociation rates of this binding reaction can be influenced. Therefore, these systems are very suitable to select for mutated immunoglobulins that have different binding properties. The mutation of the immunoglobulins can either be obtained by random mutagenesis, or directed mutagenesis based on extensive molecular modelling and molecular dynamical studies.
mRNAs encoding heavy chains of immunoglobulins raised in *Camelidae* against transition state molecules (Lerner *et al.,* 1991 *supra)* can be obtained using standard techniques. The structural genes encoding various forms of immunoglobulins according to the invention as summarized in Table 2 can be cloned into the coat protein of bacteriophages or as fusion with the anchoring part of cell wall proteins and can be tested on the catalytic property. In this way immunoglobulins or parts thereof having catalytic properties can he determined and selected. Genes encoding these selected immunoglobulins or parts thereof can be mutated as described before and recloned in bacteriophages, but preferably cloned as chimeric cell wall bound catalysts in lower eukaryotes. By performing appropriate catalytic assays, catalytic immunoglobulins or parts thereof with improved catalytic properties can be determined and selected using standard techniques.
An important application of antibodies, especially outside the pharmaceutical industry, will be chimeric proteins consisting of the binding part of antibodies and enzymes. In this way catalytic biomolecules can be designed that have two binding properties, one of the enzyme and the other of the antibody. This can result in enzymes that have superior activity. This can be illustrated with the following examples:
a. If the substrate of the enzymic reaction is produced by an organism or an enzyme is recognized by the binding domain of the antibody, the local concentration of the substrate will be much higher than for enzymes lacking this binding domain and consequently the enzymic reaction will be improved. In fact this is a mimic of vectorial metabolism in cells (compare e.g. Mitchell, (1979) Science **206** 1148-1159);
b. If the substrate of the enzymic reaction is converted into a molecule that kills organisms, then the efficiency and specificity of killing can be increased significantly if the enzyme is equipped with an antibody binding domain that recognizes the target organism (e.g. compare Takahashi *et al*., (1993) Science **259** 1460-1463);

The invention will be illustrated by the following Examples without being limited thereto. In previously filed Unilever patent specifications several expression vectors were described, e.g. for the yeasts S. *cerevisiae*, *Kluyveromyces*, and *Hansenula*, and the mould *Aspergillus*. Examples of these publications are EP-A-0173378 (UNILEVER / Ledeboer *et al*.), EP-A-0255153, *supra,* and PCT applications WO-A-91/19782 (UNILEVER / van Gorcom *et al*.) and (not prior-published) WO-A-93/12237, *supra*. The genes encoding antibodies or (functionalized) fragments thereof according to the invention can be incorporated into the earlier described expression vectors or derivatives thereof using procedures well known to a skilled person in the art. All techniques used for the manipulation and analysis of nucleic acid materials were performed essentially as described in Sambrook *et al*. (1989) (see also ref. 23 of the above given draft publication), except where indicated otherwise.

In the description of the Examples the following endonuclease restriction sites are used:

| | | | |
|---|---|---|---|
| *Afl*II | C↓TTAAG | *Mlu*I | A↓CGCGT |
| *Bsp*HI | T↓CATGA | *Nco*I | C↓CATGG |
| *Bsp*HI | T↓CATGA | *Not* | GCIGGCCGC |
| *Bst*EII | G↓GTNACC | *Nru*I | TCG↓CGA |
| *Eag*I | C↓GGCCG | *Sal*I | G↓TCGAC |
| *Eco*RI | G↓AATTC | *Xho*I | C↓TCGAG |
| *Hin*dIII | A↓AGCTT | *Bbs*I | GAAGAC(N)₂↓ CTTCTG(N')₆↓ |

### Example 1 Construction of cassettes encoding V_{H} fragments originating from Camelidae.

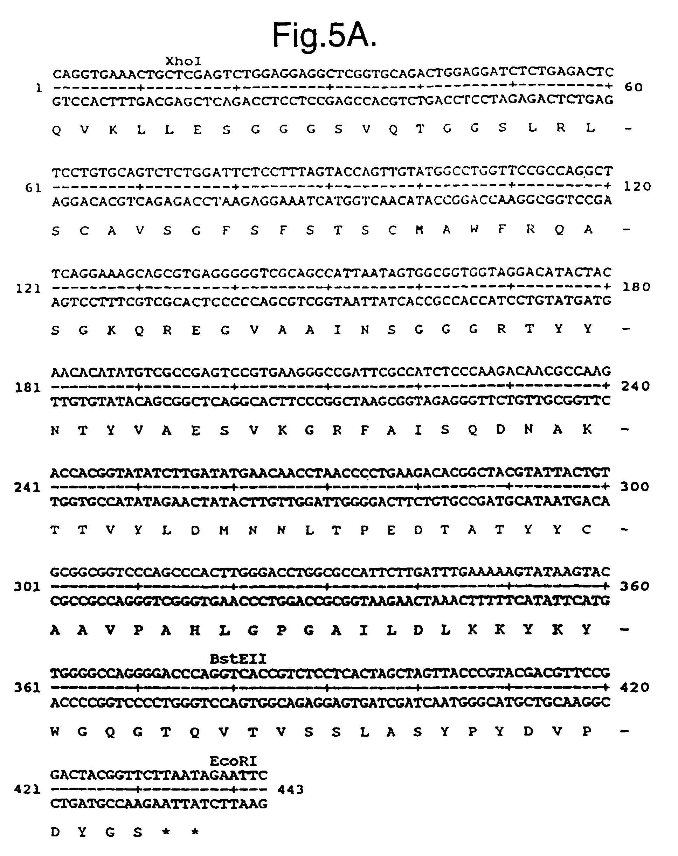

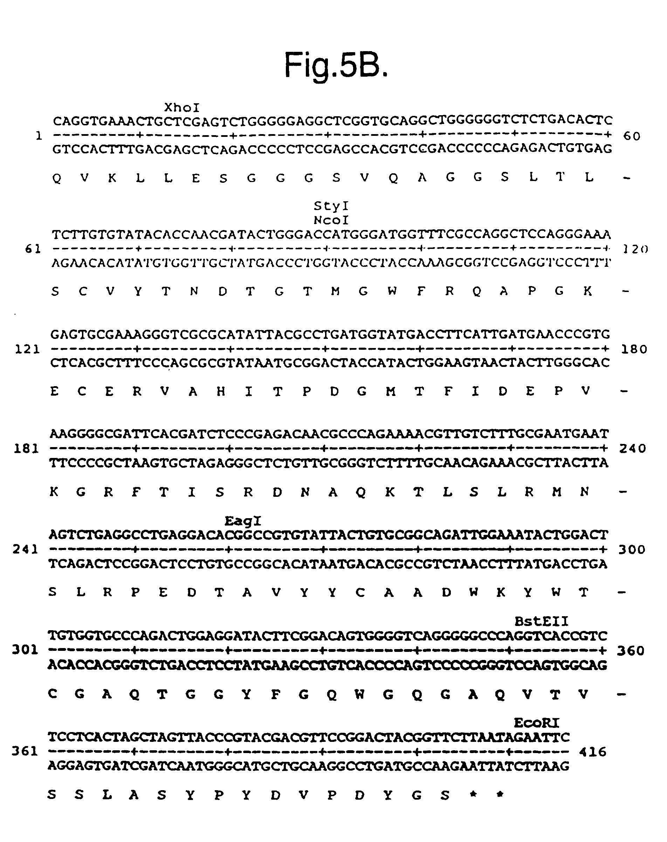

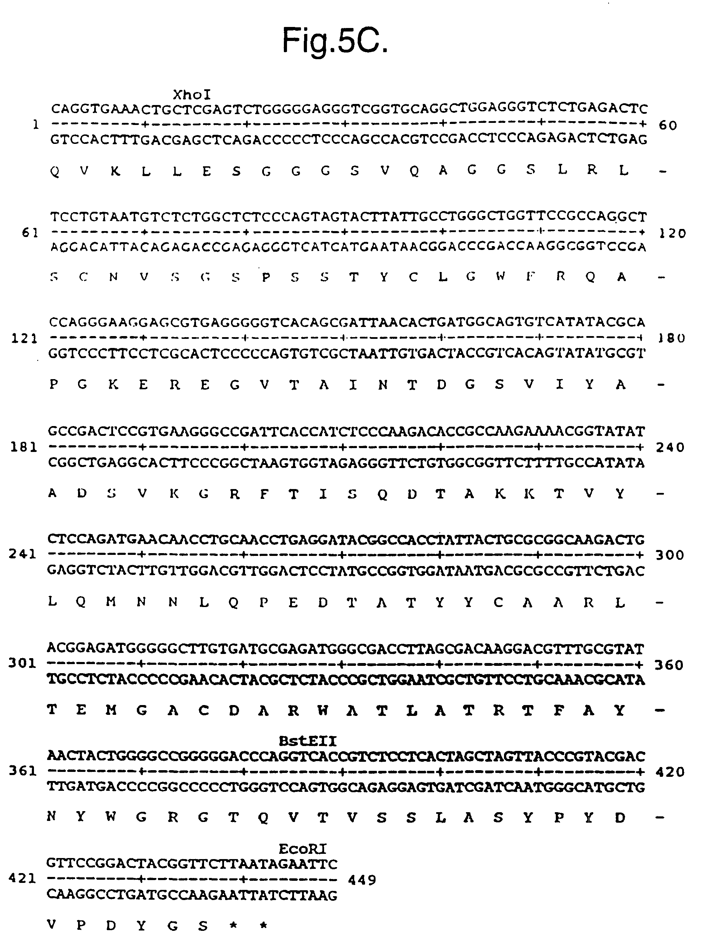

For the production of V_{H} fragments originating from *Camelidae*, the antibody gene fragments were isolated and cloned as described above in the draft publication. The thus obtained gene fragments encode the V_{H} region, a short or a long hinge region and about 14 amino acids of the C_{H}2 region. By using standard molecular biological techniques (e.g. PCR technology), the V_{H} gene fragments could be subcloned and equipped at their 5'-ends with a gene fragment encoding the *pelB* signal sequence and at their 3'-ends with a gene fragment encoding the Flag tail (13 amino acids). Three of these clones were named pB3, pB9 and pB24 and were deposited at the Centraal Bureau voor Schimmelcultures, Baarn on 20 April 1993 with deposition numbers: CBS 270.93, CBS 271.93 and CBS 272.93, respectively. The DNA and amino acid sequences of the *Camelidae*-V_{H} fragments followed by the Flag sequence are presented in Figure 5(A-C); see SEQ. ID. NO: 32-37.

### 1.1 Construction of pUR4421

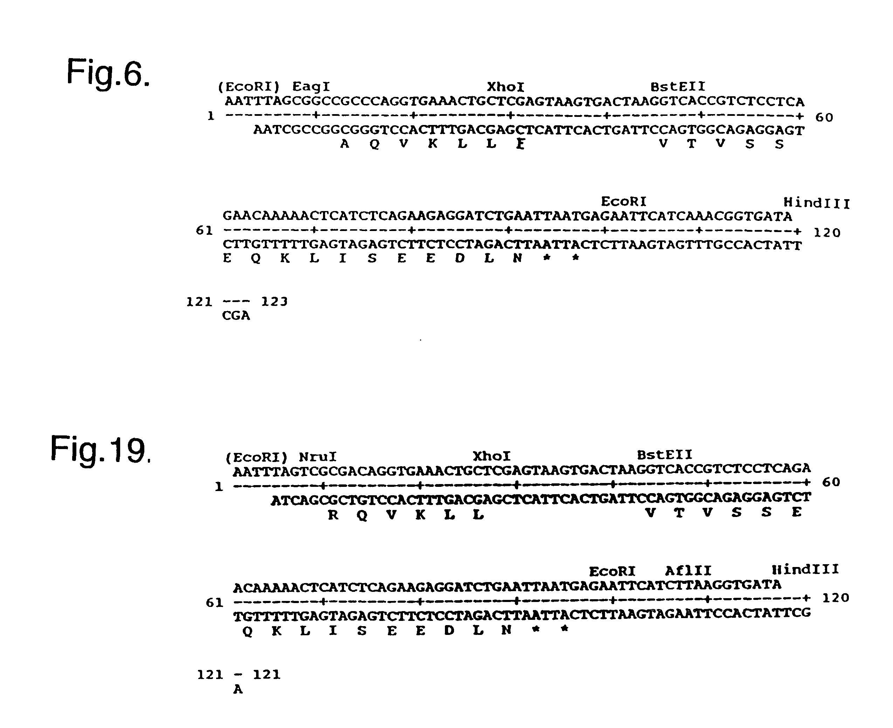

For the construction of yeast expression plasmids encoding the V_{H} fragments preceded by the invertase (=SUC2) signal sequence, the α-mating factor prepro-sequence, or the inulinase signal sequence and followed by either nothing, or a Myc tail or Flag tail, the constructs described below can be prepared.
The multiple cloning site of plasmid pEMBL9 (Denthe *et al*., 1983) (ranging from the *Eco*RI to the *Hin*dIII site) was replaced by a synthetic DNA fragment having the nucleotide sequence as indicated in Figure 6; see SEQ. ID. NO: 38-41. The 5'-part of this nucleotide sequence comprises an *Eag*I site, the first 4 codons of the *Camelidae* V_{H} gene fragment and a *Xho*I site coinciding with codons 5 and 6. The 3'-part comprises the last 5 codons of the *Camelidae* V_{H} gene (encoding VTVSS; see SEQ. ID. NO: 47) part of which coincides partially with a *Bst*EH site), eleven codons of the Myc tail, and an *Eco*RI site. The *Eco*RI site, originally present in pEMBL9, is not functional any more, because the 5'- end of the nucleotide sequence contains AATTT instead of AATTC, indicated in Figure 6 as "(*Eco*RI)". The resulting plasmid is called pUR4421.

### 1.2 Constructs with Flag tail.

After digesting the plasmid pB3 with *Xho*I and *Eco*RI, a DNA fragment of approximately 425 bp was isolated from agarose gel. This fragment codes for a truncated V_{H}-Flag fragment, missing the first 5 amino acids of the *Camelidae* V_{H}. The obtained fragment can be cloned into pUR4421. To this end plasmid pUR4421 can be digested with *Xho*I and *Eco*RI, after which the about 4 kb vector fragment can be isolated from an agarose gel. Ligation with the about 425 bp fragment will result in plasmid pUR4421-03F.

### 1.3 Constructs with Myc tail.

After digesting the plasmid pB3 with *Xho*I and *BstE*II, a DNA fragment of approximately 365 bp was isolated from agarose gel. This fragment codes for a truncated V_{H} fragment, missing both the first 4 (QVKL; see SEQ. ID. NO: 46) and the last 5 (VTVSS; see SEQ. ID. NO: 47) amino acids of the *Camelidae* V_{H} fragment.

The obtained fragment was cloned into pUR4421. To this end plasmid pUR4421 was digested with *Xho*I and *Bst*EII, after which the about 4 kb vector fragment was isolated from an agarose gel. Ligation with the about 365 bp fragment resulted in
plasmid pUR4421-03M, in which the gene encoding the V_{H} fragment is reconstituted.

### 1.4 Constructs encoding V_{H} only.

Upon digesting pUR4421-03M or pUR4421-03F with *BstE*II and *Hin*dIII, the vector fragments of about 4.4 kb can he isolated from agarose gel and religated in the presence of a synthetic linker peptide having the following sequence:

In the thus obtained plasmid, pUR4421-03, the Myc tail or Flag tail sequences are removed and the V_{H} gene fragment is directly followed by a stop codon.

### 1.5 Other constructs.

After isolating the gene fragments encoding V_{H}-hinge-C_{H}2 fragments as described above in the draft publication, or encoding the intact heavy chain immunoglobulin, it is possible, e.g. by using PCR technology, to introduce an appropriate restriction enzyme recognition site (e.g. *Eco*RI or *Hin*dIII) downstream of the hinge region, downstream of the C_{H}2 region, or downstream of the total gene. Upon isolating a *Xho*I*-Eco*RI or *Xho*I*-Hin*dIII fragment encoding the V_{H} fragment with a C-terminal extension, the fragment can be cloned into pUR4421 digested with the same restriction enzymes.

In analogy with the construction of pUR4421-03, a number of other constructs can be produced encoding functionalized heavy chain fragments in which a second polypeptide is fused to the C-terminal part of the V_{H} fragment. Optionally, the V_{H} fragment and the second polypeptide, e.g. an enzyme, might be connected to each other by a peptide linker.

To this end either the *Bst*EII-*Hin*dIII fragment or the *Bst*EII-*Eco*RI fragment of either pUR4421-03F or pUR4421-03M has to be replaced by another *Bst*EII-*Hin*dIII or *Bst*EII-*Eco*RI fragment. The latter new fragment should code for the last amino acids (VTVSS, see SEQ.ID. NO: 47) of the V_{H} fragment, optionally for a linker peptide, and for the polypeptide of interest e.g. an enzyme. Obviously, the introduction of the DNA fragment should result in an in frame fusion between the V_{H} gene fragment and the other DNA sequence encoding the polypeptide of interest.

Alternatively, it is possible to replace the *Eag*I-*Xho*I fragment of pUR4421-03 with another DNA fragment, coding for a polypeptide of interest, optionally for a peptide linker, and for the first 4 (QVKL, see SEQ.ID. NO: 46) amino acids of the V_{H} fragment, resulting in an in frame fusion with the remaining part of the V_{H} fragment. In this way, it is possible to construct genes encoding functionalized V_{H} fragments in which the second polypeptide is fused at the N-terminal part of the V_{H} fragment, optionally via a peptide linker.
Obviously, it is also possible to construct genes encoding functionalized V_{H} fragments having a polypeptide fused to the N-terminal as well as fused to the C-terminal end, by combining the above described construction routes.
The polypeptides used to functionalize the V_{H} fragments might be small, like the Myc and the Flag tails, or intact enzymes, like glucose oxidase, or both.

From all the above described constructs, derived from pUR4421, an appropriate *Eag*I*-Hin*dIII fragment, encoding the functionalized V_{H} fragment, can be isolated and cloned into a number of different expression plasmids. Several are exemplified in more detail in the following Examples. Although only the V_{H} fragments are exemplified, similar constructs can be prepared for the production of larger heavy chain fragments (e.g. V_{H}-hinge or V_{H}-hinge-C_{H}2) or intact heavy chains. The *Eag*I site is introduced before the first codon of the V_{H} fragment, facilitating an in frame fusion with different yeast signal sequences.
In particular cases, were additional *Eag*I and/or *Hin*dIII sites are present in the cloned fragments, it is necessary to perform partial digestions with one or both restriction enzymes.

Although the above and following constructions only consider the V_{H} fragment cloned in pB3, a comparable construction route can he used for the construction of expression plasmids for the production of V_{H} fragments like V_{H}-09 and V_{H}-24, or other V_{H} fragments.

### Example 2 Construction of S. cerevisiae episomal expression plasmids for Camelidae V_{H}.

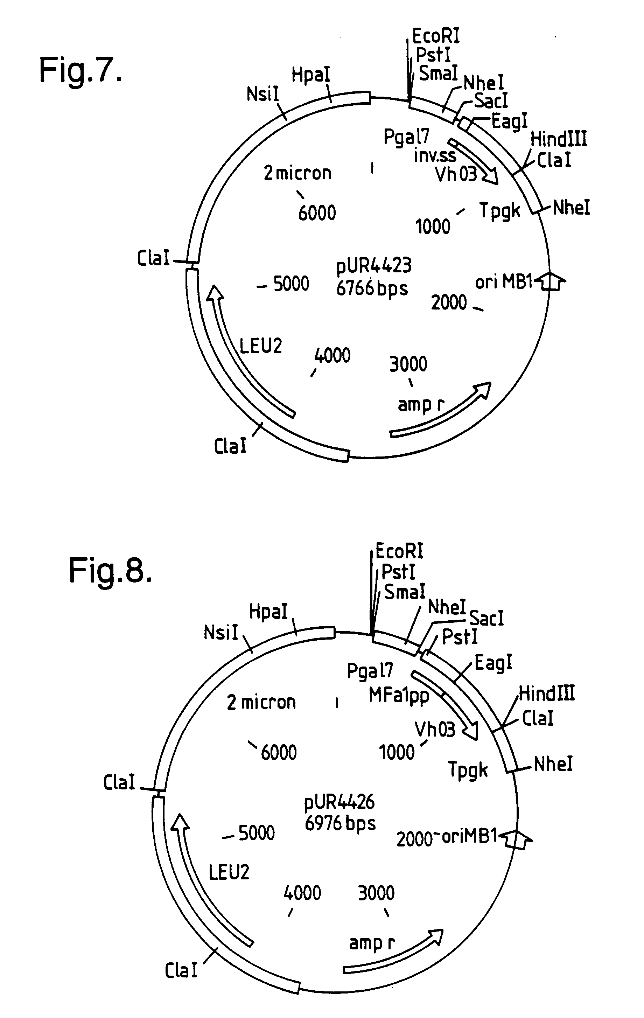

For the secretion of recombinant protein from *S*. *cerevisiae* it is worthwhile to test in parallel the two most frequently applied homologous signal sequences, the SUC2 invertase signal sequence and the prepro-α mating factor sequence.
The episomal plasmid pSY1 and pSY16 (Harmsen *et al*., 1993) contain expression cassettes for the α-galactosidase gene. Both plasmids contain the GAL7 promoter and PGK terminator sequences. pSY1 contains the invertase (SUC2) signal sequence and pSY16 contains a slightly modified (Harmsen *et al*., 1993) prepro-α-mating factor signal sequence.
Both plasmids, pSY1 and pSY16 can be digested with *Eag*I and *Hin*dIII, the about 6500 bp long vector backbone of both plasmids can be isolated and subsequently ligated with the *Eag*I/*Hin*dIII fragments from pUR4421-03F (-465 bp), pUR4421-03M (⁻455 bp) or pUR4421-03 (⁻405 bp) (See above).
This results in a series of 6 different episomal plasmids for expression in *S*. *cerevisiae*, containing behind the SUC2- and the α mating factor prepro-sequence the V_{H}-Flag coding sequence (designated pUR4423F and pUR4426F), the V_{H}-Myc coding sequence (designated pUR4423M and pUR4426M) or the coding sequence of V_{H} followed by a stop codon (designated pUR4423, Figure 7 and pUR4426, Figure 8).

Obviously, it is possible to use promoter systems different from the inducible GAL7 promoter, e.g. the constitutive GAPDH promoter.

### 2.1 Production of V_{H}-03-myc and V_{H}-24-myc.

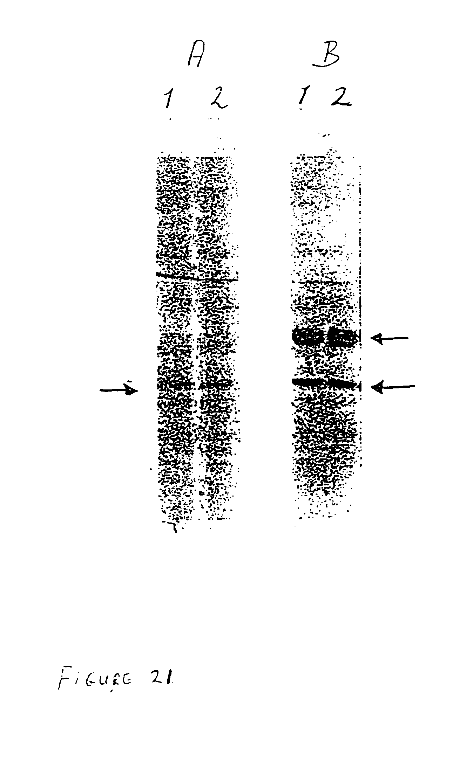

After introducing the expression plasmids pUR4423M (coding for V_{H}-03-myc, preceded by the SUC2-signal sequence) and pUR4425M (coding for V_{H}-24-myc, preceded by the SUC2-signal sequence) into S. *cerevisiae* via electroporation, transformants were selected from minimal medium agar plates (comprising 0.7 % yeast nitrogen base, 2 % glucose and 2 % agar, supplemented with the essential amino acids and bases).
For the production of antibody fragments the transformants were grown overnight in selective minimal medium (comprising 0.7 % yeast nitrogen base, 2 % glucose, supplemented with the essential amino acids and bases) and subsequently diluted ten times in YPGal medium (comprising 1 % yeast extract, 2 % bacto pepton and 5 % galactose). After 24 and 48 hours of growth, samples were taken for Western blot analysis (Figure 21). For the immuno detection of the produced V_{H}-myc fragments monoclonal anti-myc antibodies were used.

In essentially the same way comparable results were obtained with a yeast transformed with pUR4424M containing a DNA sequence encoding the V_{H}-09-myc protein.

### Example 3 Construction of S. cerevisiae multicopy integration vectors for the expression of Camelidae V_{H}.

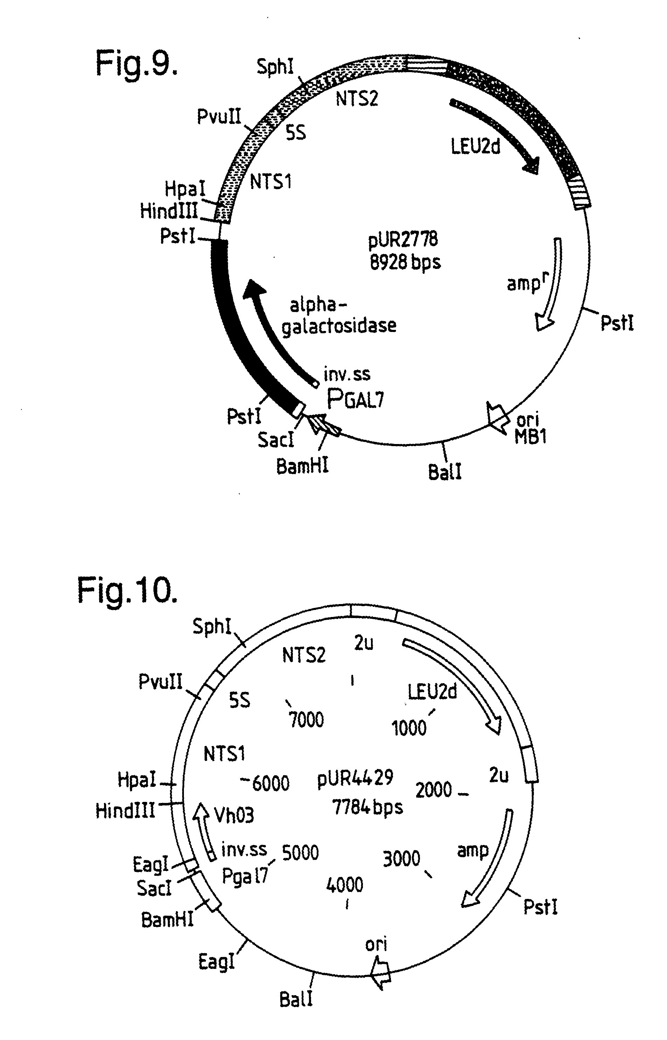

To combine the benefits of high copy number and mitotically stable expression, the concept of a multicopy integration system into the rDNA locus of lower eukaryotes has already been successfully proven (Giuseppin *et al*. *supra).*
One of these vectors is pUR2778, a derivative of pUR2774 (Giuseppin *et al*. *supra)* from which the pol1-S.O. reporter gene sequence was removed (Figure 9).
This integrating plasmid, pUR2778, can be used for integration of *Camelidae* V_{H} coding sequences, hence the vector can be digested with *Sac*I and *Hin*dIII after which the ^{~}7.3 kb vector fragment can be isolated.
From the in example 2 described pUR4423 or pUR4426 types of plasmids, *Sac*I-*Hin*dIII fragments can be isolated encoding a V_{H} fragment preceded by a signal sequence (SUC2 or α mating factor prepro) and followed by nothing or a Myc or Flag tail.
Ligation of these *Sac*I*-Hin*dIII fragments with the ⁻7.3 kb vector fragment will result in integration plasmids, encoding the (functionalized) V_{H} fragments under the regulation of the strong and inducible GAL7 promoter.
In this way the following expression plasmids were obtained:

| | |
|---|---|
| pUR4429 | P_{gal7} - SUC2 sig.seq. - V_{H}-03 |
| pUR4429F | F_{gal7} - SUC2 sig.seq. - V_{H}-03 - Flag tail |
| pUR4429M | P_{gal7} - SUC2 sig.seq. - V_{H}-03 - Myc tail |
| pUR4430 | P_{gal7} - α mat.fac. prepro. - V_{H}-03 |
| pUR4430F | P_{gal7} - α mat.fac. prepro. - V_{H}-03 - Flag tail |
| pUR4430M | P_{gal7} - α mat.fac. prepro. - V_{H}-03 - Myc tail |

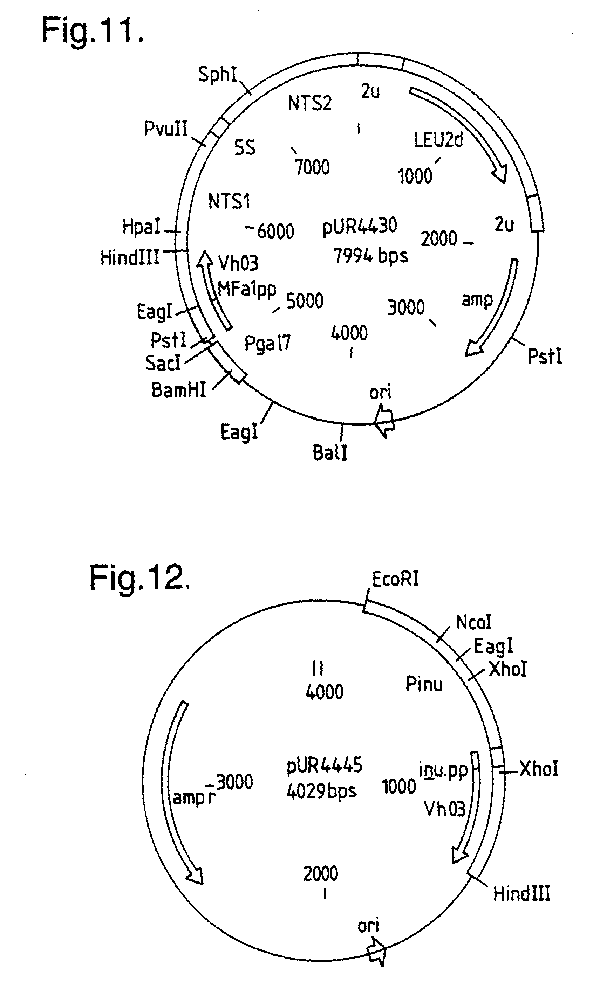

For schematic drawings see Figure 10 for pUR4429 and Figure 11 for pUR4430. Obviously, comparable constructs can he prepared for other heavy chain antibodies or fragments thereof.
As mentioned before, different promoters might be used, for example, the constitutive GAPDH promoter.

### Example 4 Construction of expression plasmids for the production of (functionalized) V_{H} fragments from Camelidae by Kluyveromyces

### 4.1. Construction of Kluyveromyces lactis episomal expression plasmids Camelidae.

Yeast strains of the genus *Kluyveromyces* have been used for the production of enzymes, such as β-galactosidase for many years, and the growth of the strains has been extensively studied. *Kluyveromyces lactis* is well known for the ability to utilize a large variety of compounds as carbon and energy sources for growth. Since these strains are able to grow at high temperatures and exhibit high growth rates, they are promising hosts for industrial production of heterologous proteins (Hollenberg, C. *et al*., EP-A-0096430, GIST-BROCADES N.V., 1983).

The plasmids pUR2427 and pUR2428 are pTZ19R derivatives with the promoter and the DNA sequence encoding either the signal peptide (=pre-sequence) (in pUR2428), or the natural prepro-sequence (in pUR2427), of inulinase (inu) from *Kluyveromyces marxianus*. Both plasmids contain a unique *Bsp*MI site suitable to create a perfect joint with *Eag*I or *Not*I digested DNA-fragments (not yet published European patent application 92203932.6, *supra*). In both plasmids a unique *Hin*dIII site is located a bit further downstream of the *Bsp*MI-site, so that *Eag*I-*Hin*dIII cut DNA-fragments encoding V_{H} from *Camelidae* either solely or with Myc- or Flag- tail can he easily ligated into *Bsp*MI-*Hin*dIII digested pUR2427 or pUR2428. Thereby a set of six plasmids can he created containing the promoter and secretion signals of the *Kluyveromyces marxianus* inulinase gene, joint in frame to *Camelidae* Vh encoding sequences, all on a *Eco*RI-*Hin*dIII restriction fragment:

| | |
|---|---|
| pUR4445 | Pᵢₙᵤ - lnu prepro seq. - V_{H} - 03 |
| pUR4445M | Pᵢₙᵤ - Inu prepro seq. - V_{H} - 03 - Myc |
| pUR4445F | Pᵢₙᵤ - Inu prepro seq. - V_{H} - 03 - Flag |
| pUR4446 | Pᵢₙᵤ - Inu pre seq. - V_{H} - 03 |
| pUR4446M | Pᵢₙᵤ - Inu pre seq. - V_{H} - 03 - Myc |
| pUR4446F | Pᵢₙᵤ - Inu pre seq. - V_{H} - 03 - Flag. |
| Maps of pUR4445 and pUR4446 are shown in Figure 12 and Figure 13, | |

The *Eco*RI*-Hin*dIII fragments of these plasmids can be ligated into the expression vector pSK1 (not yet published European patent application 92203932.6, *supra),* from which the α-galactosidase expression cassette including the *GAL7*-promoter is removed with a *Eco*RI(partial) and *Hin*dIII digestion. The resulting plasmids can then be transformed for example in *K*. *lactis* strain MSK110 (a, *uraA*, *trp1*::*URA3*), as they contain the trp1 marker and the pKD1 episomal plasmid sequences:

| | |
|---|---|
| pUR4447 | Pᵢₙᵤ - Inu prepro seq. - V_{H} - 03 |
| pUR4447M | Pᵢₙᵤ - Inu prepro seq. - V_{H} - 03 - Myc |
| pUR4447F | Pᵢₙᵤ - Inu prepro seq. - V_{H} - 03 - Flag |
| pUR4448 | Pᵢₙᵤ - Inu pre seq. - V_{H} - 03 |
| pUR4448M | Pᵢₙᵤ - Inu pre seq. - V_{H} - 03 - Myc |
| pUR4448F | Pᵢₙᵤ - Inu pre seq. - V_{H} - 03 - Flag . |
| A map of pUR4447 is shown in Figure 14. | |

Transformation can he performed by standard techniques such as the methods of Beggs (1978) or electroporation, using 0.67% Yeast Nitrogen Base (without amino acids) and 2% glucose as the selection medium for transformants.

### 4.2. Construction of Kluyveromyces lactis multicopy integration vectors.

Alternatively, since all tailed and non-tailed versions of the Vh fragments, joined to the inulinase promoter and secretion signals, are located on *Eco*RI-*Hin*dIII fragments, the rDNA multicopy integration plasmid pMIRKGAL-TΔ1 (Bergkamp *et al*., 1992) can be used in a similar way as the pSK1 plasmid. In order to replace the α-gal expression cassette present in this plasmid, by a antibody fragment cassette, these plasmids have to he digested with *Eco*RI(partial) and *Hin*dIII. After isolating the vector fragments, they can he ligated with the about 1.2 kb *Eco*RI-*Hin*dIII fragments which can he obtained from the plasmids described in example 4.1. The resulting plasmids can he linearized with *Sac*II and transformed to MSK110, resulting in *K*. *lactis* strains with potentially high and stable expression of single chain V_{H} fragments.

| | |
|---|---|
| pUR4449 | Pᵢₙᵤ - Inu prepro seq. - V_{H} - 03 |
| pUR4449M | Pᵢₙᵤ - Inu prepro seq. - V_{H} - 03 - Myc |
| pUR4449F | Pᵢₙᵤ - Inu prepro seq. - V_{H} - 03 - Flag |
| pUR4450 | Pᵢₙᵤ - Inu pre seq. - V_{H} - 03 |
| pUR4450M | Pᵢₙᵤ - Inu pre seq. - V_{H} - 03 - Myc |
| pUR4450F | Pᵢₙᵤ - Inu pre seq. - V_{H} - 03 - Flag . |

### 4.3. Construction of Kluyveromyces marxianus episomal plasmids.

*Kluyveromyces marxianus* is a yeast which is perhaps even more attractive than K *lactis* for industrial biotechnology, due to its short generation time on glucose (about 45 minutes) and its ability to grow on a wide range of substrates, and its growth at elevated temperatures (Rouwenhorst *et al*., 1988).
The shuttle vector pUR2434, containing the leu2 marker and the pKD1 plasmid sequences (not yet published European patent application 92203932.6, *supra*), located on a pUC19 based vector, can be cut with *Eco*RI(partial) and *Hin*dIII to remove the α-galactosidase expression cassette. In this vector the *Eco*RI-*Hin*dIII fragments containing the Vh expression cassettes as described in example 4.1, can be ligated. The resulting plasmids can then he transformed into KMS3, the neat leu2-auxotroph CBS6556 *K*. *marxianus* strain (Bergkamp, 1993) using the method of Meilhoc *et al*. (1990).

| | |
|---|---|
| pUR4451 | Pᵢₙᵤ - Inu prepro seq. - V_{H} - 03 |
| pUR4451M | Pᵢₙᵤ - Inu prepro seq. - V_{H} - 03 - Myc |
| pUR4451F | Pᵢₙᵤ - Inu prepro seq. - V_{H} - 03 - Flag |
| pUR4452 | Pᵢₙᵤ - Inu pre seq. - V_{H} - 03 |
| pUR4452M | Pᵢₙᵤ - Inu pre seq. - V_{H} - 03 - Myc |
| pUR4452F | Pᵢₙᵤ - Inu pre seq. - V_{H} - 03 - Flag . |
| A map of pUR4451 is shown in Figure 15. | |

### 4.4 Construction of Kluyveromyces marxianus multicopy integration vectors.

For high and stable expression in *Kluyveromyces marxianus*, the multicopy integration system as described by Bergkamp (1993), can be used. The following cloning route, based on the route for constructing pMIRKM-GAL5 (Bergkamp, 1993), results in suitable expression vectors for production of Vh fragments from *Camelidae*. The *Eco*RI*-Nhe*I(Klenow filled) fragments of pUR4447,-M,-F and pUR4448,-M,-F containing the Vh fragment expression cassettes as described in example 4.1, can be isolated and ligated in *Eco*RI-*Eco*RV digested pIC-20H. From the plasmids obtained in this way, and which are equivalents of the pIC-αgal plasmid, the *Bam*HI*-Nru*I fragment can be isolated and ligated with *Bam*HI*-Sma*I digested pMIRKM4. The result of this will be expression vectors which are equivalent to pMIRKM-GAL5, and contain a tailed or non-tailed Vh fragment from camel under control of inulinase promoter and secretion signals, in a vector which also contains the K *marxianus LEU2*-gene with defective promoter, and *K*. *marxianus* rDNA sequences for targeted integration into the genome. These vectors can be used to transform for example KMS3.

| | |
|---|---|
| pUR4453 | Pᵢₙᵤ - Inu prepro seq. - V_{H} - 03 |
| pUR4453M | Pᵢₙᵤ - Inu prepro seq. - V_{H} - 03 - Myc |
| pUR4453M | Pᵢₙᵤ - Inu prepro seq. - V_{H} - 03 - Flag |
| pUR4454 | Pᵢₙᵤ - Inu pre seq. - V_{H} - 03 |
| pUR4454M | Pᵢₙᵤ - Inu pre seq. - V_{H} - 03 - Myc |
| pUR4454F | Pᵢₙᵤ - Inu pre seq. - V_{H} - 03 - Flag . |
| A map of pUR4453 is shown in Figure 16. | |

### Example 5. Construction of Hansenula polymorpha integrating vectors for the expression of (functionalized) V_{H} fragments from Camelidae.

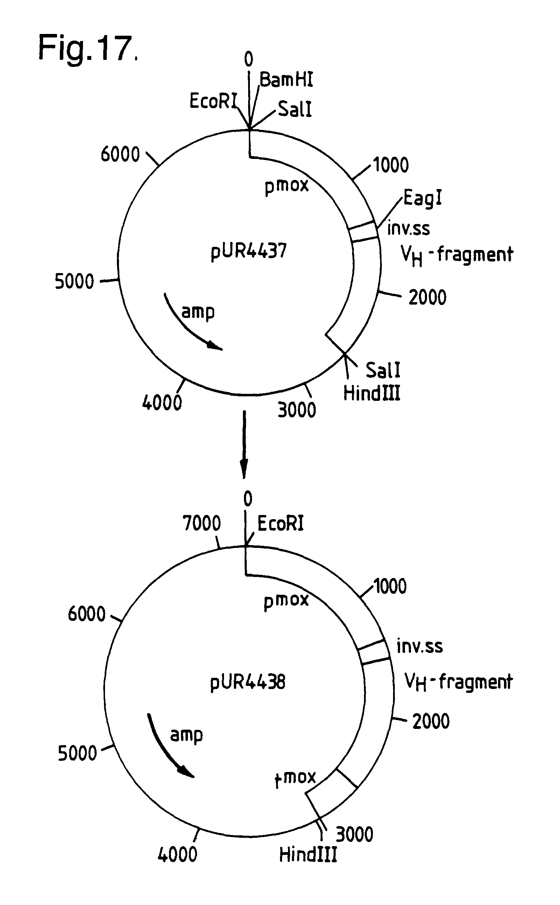

In search for productive systems able to carry out authentic posttranscriptional processing and overcoming the limitation of higher eukaryotic expression systems, such as high costs, low productivity and the need for stringent control procedures for the detection of contaminating agents could be overcome by the methylotrophic yeast *H*. *polymorpha*. This strain is able to grow on methanol as its sole carbon and energy source, so the presence of methanol in the growth medium rapidly induces the enzymes of the methanol pathway, such as the key enzymes methanol oxidase (MOX) and dihydroxyacetone synthase (DHAS).
While experiments to express foreign genetic information from an episomal plasmid resulted a low plasmid stability, chromosomal integration is the method of choice (Sierkstra *et al*., 1991). By utilizing the DNA of the *mox* gene as integration locus the latter were able to express and secrete α-galactosidase regulated by *mox* promoter and -terminator. Here, the *S*. *cerevisiae* SUC2 signal sequence was proven to be efficiently functional for secretion.
The same approach can be used for expression and secretion of *Camelidae* V_{H} antibody fragments. Plasmids analogous to pUR3515 (without an origin of replication functional in yeast) and pUR3517 (containing the HARS2 sequence as origin of replication) can be used as expression vectors (Sierkstra *et al*., 1991). As a starting vector pUR3501 can be used (Sierkstra *et al*., 1991) in which by means of site directed mutagenesis (e.g. via PCR technology), an *Eag*I restriction site is introduced at the junction between the invertase (=SUC2) signal sequence and the α-galactosidase. From the resulting plasmid, pUR3501*Eag*, it is possible to replace the *Eag*I-*Hin*dIII fragment comprising the α-galactosidase gene by an *Eag*I-*Hin*dIII fragment encoding a (functionalized) antibody fragment, obtained as described in example 1. In case of using the *Eag*I-*Hind*III fragments of the pUR4421-03 series (example 1), this would result in plasmids pUR4437 (Figure 17), pUR4437M and pUR4437F. In these plasmids the nucleotide sequence encoding the (functionalized) V_{H} is preceded by a nucleotide sequence encoding the invertase signal sequence and the *mox* promoter sequence. The obtained plasmids can he digested with *Bam*HI and *Hin*dIII and after filling in the sticky ends with Klenow polymerase, the about 2.6 kb fragments can he ligated into plasmid pUR3511 which was digested with

*Sma*I (Sierkstra *et al*., 1991). In this way the terminator sequence of the *mox* gene can by fused downstream of the V_{H} encoding sequences. From the thus obtained plasmids, pUR4438 (Figure 17) *Eco*RI-*Hin*dIII fragments of about 3 kb can be isolated, containing the *mox* promoter, the invertase signal sequence, the (functionalized) V_{H} fragment and the *mox* transcription terminator. Subsequently these fragments can be cloned into plasmid pUR3513 (no yeast origin of replication) or in pUR3514 (HARS origin of replication) as described by Sierkstra *et al*. (1991), resulting in two sets of plasmids:

| | |
|---|---|
| pUR4439 | Pₘₒₓ - SUC2 sig. seq. - V_{H} - *mox* term. -- no origin |
| pUR4439M | Pₘₒₓ - SUC2 sig. seq. - V_{H} - *mox* term. -- no origin |
| pUR4439F | Pₘₒₓ - SUC2 sig. seq. - V_{H} - *mox* term. -- no origin |
| pUR4440 | Pₘₒₓ - SUC2 sig. seq. - V_{H} - *mox* term. -- HARS origin |
| pUR4440M | Pₘₒₓ - SUC2 sig. seq. - V_{H} - *mox* term. -- HARS origin |
| pUR4440F | Pₘₒₓ - SUC2 sig. seq. - V_{H} - *mox* term. -- HARS origin . |
| Maps of pUR4439 and pUR4440 are shown in Figure 18. | |

Essentially the same can be done with other *Eag*I-*Hin*dIII fragment, obtained as described in example 1.
The newly obtained plasmids can he transformed by electroporation of H. *polymorpha* A16 (CBS4732, *leu*-) and can be selected by growing on selective medium containing 0.68% YNB and 2% glucose. Induction medium should contain 0.5% methanol instead of the glucose.

### Example 6 Construction Aspergillus niger var. awamori integration vectors for the production of V_{H} fragments from Camelidae.

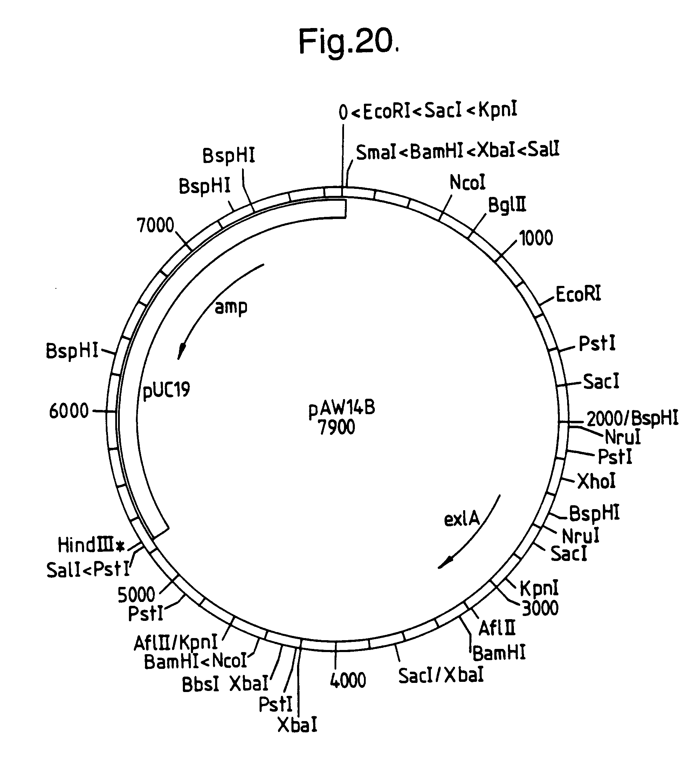

The multiple cloning site of plasmid pEMBL9 (ranging from the *Eco*RI to the *Hin*dIII site) was replaced by a synthetic DNA fragment having the nucleotide sequence as indicated in Figure 19; see SEQ. ID. NO: 42-45. The 5'- part of the nucleotide sequence contains a *Nru*I restriction site followed by the first codons of the *Camelidae* V_{H} gene fragment and a *Xho*I restriction site. The 3'-part encodes for a *Bst*EII restriction site, the last codons of the *Camelidae* V_{H} gene, eleven codons of the Myc tail and finally a *Eco*RI and a *Afl*II site. The resulting plasmid is pUR4432.

After digesting plasmid pB3 with *Xho*I and *Eco*RI, *a* DNA fragment of approximately 425 bp can be isolated from agarose gel. This fragment codes for a truncated V_{H}-Flag fragment, missing the first 5 amino acids of the *Camelidae* V_{H}.
The obtained fragment can he cloned into pUR4432. To this end plasmid pUR4432 can be digested with *Xho*I and *Eco*RI, after which the about 4 kb vector fragment was isolated from an agarose gel. Ligation with the about 425 bp fragment resulted in plasmid pUR4433F.

After digesting the plamids pB3 with *Xho*I and *Bst*EII, a DNA fragment of approximately 365 bp was isolated from agarose gel. This fragment codes for a truncated V_{H} fragments, missing the first and last 5 amino acids of the *Camelidae* V_{H}.
The obtained fragment was cloned into pUR4432. To this end plasmids pUR4432 can be digested with *Xho*I and *BstE*II, after which the about 4 kb vector fragment was isolated from an agarose gel. Ligation with the about 365 bp fragments resulted in plasmids pUR4433M. In a similar way the *Xho*I-*Bst*EII fragments of pB9 and pB24 were cloned into the pUR4432 vector fragment, resulting in pUR4434M and pUR4435M, respectively.
Upon digesting pUR4433M or pUR4433F with *BstE*II and *Hin*dIII, the vector fragments of about 4.4 kb can be isolated from agarose gel and religated in the presence of a synthetic linker peptide having the following sequence:

In the thus obtained plasmid, pUR4433, the Myc tail or Flag tail sequences are removed and the V_{H} gene fragment is directly followed by a stop codon.

Analogous as described in example 1.5, it is possible to clone nucleotide sequences encoding longer fragments of the heavy chain immunoglohulins into pUR4432 or to replace the *BstE*II-*Afl*II fragments of the above mentioned plasmids pUR4433, pUR4433F or pUR4433M with other *BstE*II-*Afl*II fragments, resulting in frame fusions encoding functionalized V_{H} fragments, having a C-terminal extension.

Upon replacing the *Nru*I-*Xho*I fragments of pUR4433, pUR4433F or pUR4433M, in frame fusions can be constructed encoding functionalized V_{H} fragments, having an N-terminal extension.
In the above described constructs an *Nru*I site was introduced before the first codon of the (functionalized) V_{H} fragment, facilitating an in frame fusion with the precursor-sequence of xylanase, see (not prior-published) WO-A-93/12237, *supra.*
For the construction of *Aspergillus* expression plasmids, from the plasmids pUR4433F, pUR4433M and pUR4433, respectively, an about 455, 445 and 405 bp *Nru*I-*Afl*II fragment has to be isolated encoding the V_{H} fragment with a Flag, a Myc or no tail.

Plasmid pAW14B was the starting vector for construction of a series of expression plasmids containing the *exlA* expression signals and the genes coding for (functionalized) V_{H} fragments of *Camelidae* heavy chain antibodies. The plasmid comprises an *Aspergillus niger* var. *awamori* chromosomal 5 kb *Sal*I fragment on which the 0.7 kb *exlA* gene is located, together with 2.5 kb of 5'-flanking sequences and 2.0 kb of 3'-flanking sequences (see Figure 20 and (not prior-published) WO-A-93/12237, *supra).*
Starting from pAW14B, pAW14B-10 was constructed by removing the *Eco*RI site originating from the pUC19 polylinker, and introducing a *Not*I site. This was achieved by digesting plasmid pAW14B with *Eco*RI and after dephosphorylation the linear 7.9 kb *Eco*RI fragment was isolated. The fragment was religated in the presence of the "*Eco*RI"-*Not*I linker: Subsequently the *Afl*II site, located downstream of the *exlA* terminator was removed by partially cleaving plasmid pAW14B-10 and religating the isolated, linearized plasmid after filling in the sticky ends, resulting in plasmid
pAW14B-11.
Finally, pAW14B-12 was constructed using pAW14B-11 as starting material. After digestion of pAW14B-11 with *Afl*II (overlapping with the *exlA* stop codon) and *Bgl*II (located in the *exl* promoter) the ^{~}2.4 kb *Afl*H-*Bgl*H fragment, containing part of the *exlA* promoter and the *exlA* gene was isolated as well as the ^{~}5.5 kb *Afl*II*-Bgl*II vector fragment. After partial digestion of this ^{~}2.4 kb fragment with *Bsp*HI (located in the *exl*A promoter and at the *exlA* start codon) an about 1.8 kb *Bgl*II-*Bsp*HI *exlA* promoter fragment (up to the ATG initiation codon) was isolated and ligated with the about 5.5 kh *Afl*H-*Bgl*H vector fragment of pAW14B-11 in the presence of the following adaptor:

For the construction of the V_{H} expression plasmids, pAW14B-11 can be partially digested with *Nru*I and digested with *Afl*II, after which the ^{~} 7 kb vector fragment can be isolated from agarose gel and contains the xylanase promoter, the DNA sequence encoding the xylanase signal sequence and the xylanase terminator. Upon ligation of the *Nru*I-*Afl*II fragments of pUR4433M, pUR4434M and pUR4435M with the pAW14B-11 vector, plasmids pUR4436M, pUR4437M and pUR4438M were obtained, respectively. In these plasmids the *Camelidae* V_{H} polypeptides are preceded by the 27 amino acid long precursor sequence of xylanase and followed by the myc-tail (of 11 amino acids; see Examples 1.3 en 2, Figures 6 and 19, and SEO.ID. NO: 41 = 45).
In a similar way plasmids can be constructed encoding the V_{H} fragments followed by the FLAG-tail or without a tail.
After introducing the *amdS* and *pyrG* selection markers into the unique *Not*I site of pUR4436M, pUR4437M and pUR4438M using conventional techniques, e.g. as described in Examples 2 and 3 of (not prior-published) WO-A-93/12237, *supra,* the plasmids were transferred to *Aspergillus*.
Production of the Camel V_{H} fragments by the selected transformants was achieved by growing the strains in inducing medium essentially as described in example 2.2 of (not prior-published) WO-A-93/12237, *supra*. Western blot analysis of the culture medium was perforemd as described in Example 2.1 above and revealed the presence of the antibody fragments.

Obviously, expression vectors can he constructed in which different promoter systems, e.g. glucoamylase promoter, and/or different signal sequences, e.g. glucoamylase or glucose oxidase signal sequences, are used.

### Example 7 Production of glucose oxidase - V_{H} fusion proteins

Glucose oxidase catalyses the oxidation of D-glucose to D-gluconate under the release of hydrogen peroxide. Glucose oxidase genes (*gox*) from *Aspergillus niger* have been cloned (Frederick *et al*. (1990) J. Biol. Chem. **265** 3793, Kriechbaum *et al*., 1989) and the nucleotide sequences are available from the EMBL data bank under accession numbers J05242 and X16061. The nucleotide sequence of the latter is used as a basis for the following construction route.
Upon cloning the *gox* gene from *A*. *niger* it is possible, by applying PCR technology, to introduce convenient restriction sites.
To introduce a *Bsp*HI restriction site, overlapping with the ATG initiation codon, the sequence ATC ATG CAG can he changed to ATC ATG AGG. In the same experiment an *Eco*RI restriction site can be introduced which is located upstream of the *Bsp*HI site. This can be achieved by using the following PCR primer:

A second PCR primer, having the following sequence can be used: in the same PCR experiment, in order to introduce a *Bbs*I site, a *Afl*II site and a *Hin*dIII site, downstream of the unique Sail site present in the glucose oxidase gene. After digesting the DNA obtained from this PCR experiment with *Eco*RI and *Hin*dIII, an *Eco*RI - *Hin*dIII fragment of about 160 bp can be isolated and cloned into pEMBL9, which was digested with the same enzymes, resulting in plasmid pGOX1.
From pGOX1 an about 140 bp *Bsp*HI - *Afl*II fragment can be isolated and introduced into the 7.2 kb *Bbs*I-*Afl*II vector fragment of pAW14B-12, resulting in pAW14B-GOX. In this plasmid, the 5'- part of the *gox* gene, encoding the first 43 amino acids, is fused in frame with the ATG initiation codon of the *exlA* gene.

In a second PCR experiment, a *Mlu*I restriction site can be introduced near the 3'-end of the *gox* by changing the sequence TAT GCT TCC to TAC GCG TCC. In the same experiment a *Hin*dIII site can be introduced downstream of the *Mlu*I site. As a second primer an oligo nucleotide should be used hybridizing upstream of the *Sail* site. After digesting the DNA obtained from this PCR experiment with *Sal*I and *Hin*dIII, an *Sal*I - *Hin*dIII fragment of about 1.7 kb can be isolated and cloned into pEMBL9, which was digested with the same enzymes, resulting in plasmid pGOX2. Upon digesting pGOX2 with *Mlu*I and *Hin*dIII, an about 5.7 kb vector fragment can be isolated.
From the plasmids pUR4433, pUR4433F, pUR4433M and the like, *Xho*I-*Hin*dIII fragments can be isolated, encoding the truncated *Camelidae* V_{H} fragment with or without a tail sequence, and missing the first 4-6 N-terminal amino acids (see Example 1). These fragments can be ligated into the 5.7 kb pGOX2 vector fragment by using *Mlu*I*-Xho*I adaptors. These adaptors are designed in such a way that they result in an in frame fusion between the 3'-end of the *gox* gene and the restored V_{H} gene fragment, optionally intersected with a DNA sequence encoding a peptide linker sequence.
An example of these designed adaptors is: which encodes for the last amino acids of GOX, an SSGGSS linker sequence (see SEQ. ID. NO: 62) and the N-terminal amino acids of the Camel V_{H} fragment of pB3. Instead of the SSGGSS linker (see SEQ. ID. NO: 62) it is possible to use other linkers such as the repeated sequence linkers described in the above indicated European patent application 92402326.0, e.g. a repeated sequence Pro-X, with X being any amino acid, but preferably Gln, Lys or Glu, the sequence containing advantageously at least 3 repeats of Pro-X and especially a fragment composed of a 12-fold repeat of the sequence Pro-X.

In case the about 435 bp *Xho*I-*Hin*dIII fragment of pUR4433M is used in combination with the above described adaptor, this would result in pGOX2-03M.
From this plasmid a *Sal*I-*Afl*II fragment of about 2.1 kb encoding the C-terminal part of glucose oxidase followed by the linker peptide, the Camel V_{H} fragment of pB3 and finally the Myc tail.
Upon digesting pAW14B-GOX partially with *Bbs*I, and with *Afl*II, the about 7.4 kb vector fragment can he isolated. This fragment contains the xylanase promoter, the DNA sequence encoding the N-terminal part of glucose oxidase and the xylanase promoter. Due to the digestion with *Bbs*I, a *Sal*I sticky end is created, corresponding with the *Sal*I restriction site originally present in the *gox* gene. Ligation of the *Sal*I-*Afl*II vector fragment with the about 2.1 kb *Sal*I-*Afl*II fragment of pGOX2-03M, resulting in pUR4441M. This expression plasmid encodes for a single chain polypeptide comprising the glucose oxidase enzyme, the (functionalized) Camel V_{H} fragment and the Myc tail.
Introduction of this type of expression plasmids in *Aspergillus* can be achieved essentially as described in example 6.
As the naturally occurring glucose oxidase is a homodimeric enzyme, it might be expected that a fusion protein, comprising glucose oxidase and an antibody fragment as a C-terminal extension, has an increased avidity for the antigen/antibody binding, if this fusion protein is produced as a homodimer. Alternatively, it is possible to produce heterodimers, consisting of one glucose oxidase molecule connected to a V_{H} fragment and one wild type glucose oxidase molecule. This can be achieved by producing with the same strain both wild type glucose oxidase and the fused glucose oxidase-V_{H} fragment, or by mixing the two different homodimers produced by different strains under conditions whereby the mixture of dimers are dissociated and subsequently associated.

### Example 8 Engineering of Camelidae V_{H} fragments

### 8.1 Random and targeted random mutagenesis.

After expressing a number of different *Camelidae* V_{H} fragments in lower eukaryotic host organisms as described above, or in prokaryotes, fragments produced in relative higher amounts can he selected. Upon subjecting the *Xho*I-*Bst*EII gene fragments to a (targeted) random mutagenesis procedure, it might be possible to further improve special characteristics of the V_{H} fragment, e.g. further improvement of the production level, increased stability or increased affinity.
To this end the following procedure might be followed.
Upon replacing the polylinker of the phagemid vector pHEN1 (Hoogenboom *et al*., 1991) located on a *Nco*I*-Not*I fragment by a new polylinker having the following sequence: (see SEQ. ID. NO: 60-61) it becomes possible to introduce *Xho*I-*Bst*EII fragments encoding truncated *Camelidae* V_{H} fragments in the phagemid.
Following mutagenesis of the V_{H} encoding sequence (random mutagenesis) or a specific part thereof (targeted random mutagenesis), the mutated V_{H} fragments can be expressed and displayed on the phage surface in essentially the same way as described by Hoogenboom *et al*. (1991). Selecting phages displaying (mutant) V_{H} fragments, can be done in different ways, a number of which are described by Marks *et al*. (1992). Subsequently, the mutated *Xho*I-*Bst*EII fragments can be isolated from the phagemid and introduced into expression plasmids for yeast or fungi as described in previous examples.
Upon producing the mutant V_{H} fragments by these organisms, the effects of the mutations on production levels, V_{H} fragment stability or binding affinity can be evaluated easily and improved V_{H} fragments can be selected.
Obviously, a similar route can be followed for larger antibody fragments. With similar procedures the activity of catalytic antibodies can be improved.

### 8.2 Site-directed or designed mutagenesis

As an alternative to the methods described above in Example 8.1 it is possible to use the well-known technique of site-directed mutagenesis. Thus, designed mutations, preferably based on molecular modelling and molecular dynamics, can be introduced in the V_{H} fragments, e.g. in the framework or in the CDRs.

### 8.3 Construction V_{H} fragments with regulatable binding efficiencies.

For particular applications, the possibility to regulate the binding capacity of antibody fragments might he necessary. The introduction of metal ion binding sites in proteins is known from the literature e.g. Pessi *et al*. (1993). The present inventors envisage that the introduction of a metal binding site in an antibody fragment by rational design can result in a regulatable antibody fragment, when the metal binding site is introduced at a position such that the actual binding of the metal ion results in a conformational change in the antibody fragments due to which the binding of the antigen to the antibody fragment is influenced. Another possibility is that the presence of the metal prevents antigen binding due to steric hindrance.

### 8.4 Grafting of CDR regions on the framework fragments of a Camelidae V_{H} fragment.

Grafting of CDR fragments onto framework fragments of different antibodies or fragments thereof is known from the literature (see Jones *et al*. (1986), WO-A-92/15683, and WO-A-92/01059). In these cases the CDR fragments of murine antibody fragments were grafted onto framework fragments of human antibodies. The sole rationale behind the "humanization" was to increase the acceptability for therapeutic and/or diagnostic applications in human.
Essentially the same approach can however also be used for a totally different purpose. Although antibody fragments share some homology in the framework areas, the production levels vary considerably.
Once an antibody or an antibody fragment, e.g. a *Camelidae* V_{H} fragment, has been identified, which can be produced to high levels by an production organism of interest, this antibody (fragment) can he used as a starting point to construct "grafted" antibody (fragments), which can he produced in high levels and have an other specificity as compared to the original antibody (fragment). In particular cases it might be necessary to introduce some modifications in the framework fragments as well in order to obtain optimal transitions between the framework fragments and the CDR fragments. For the determination of the optimal transitions molecular dynamics and molecular modelling can be used.
To this end a synthetic gene, encoding the "grafted V_{H}" fragment, can be constructed and introduced into an expression plasmid. Obviously it is possible to adapt the codon usage to the codons preferred by the host organism.
For optimization of the "grafted V_{H}" fragment, the procedure as described in example 8.1 can he followed.

### Literature mentioned in the specification additional to that mentioned in the above given draft publication

- Adair, J.R. *et al*., WO-A-92/01059 (CELLTECH Ltd, 1992)
- Beggs (1978) Nature **275** 104
- Bendig, M.M. *et al.* WO-A-92/15683 (MERCK PATENT GmbH, 1992)
- Bergkamp, R.J.M., Kool, I.M., Geerse, R.H., Planta, R.J. (1992) Multiple copy integration of the α-galactosidase gene from *Cyamopsis tetragonoloba* into the ribosomal DNA of *Kluyveromyces lactis*. Current Genetics **21** 365-370
- Bergkamp, R.J.M., PhD Thesis Free University of Amsterdam (1993), Heterologous gene expression in *Kluyveromyces* yeasts
- Better *et al.* (1988) Science **240** 1041-1043
- Bird et al., (1988) Science **242** 423-426
- Cabilly, S. et *al*., EP-A-0125023 (GENENTECH, 1984)
- Denthe, *et al*. (1983) Nucl. Acids Res. **11** 1645
- Fellinger, A.J. *et al*., EP-A-0255153 (UNILEVER, 1988)
- Frederick *et al*. (1990) J. Biol. Chem. **265** 3793
- Giuseppin, M.L.F., Lopes, M.T.S., Planta, R.J., Verbakel, J.M.A., Verrips, C.T. (1991) Process for preparing a protein by a yeast transformed by multicopy integration of an expression vector. PCT application WO 91/00920 (UNILEVER)
- Harmsen, M.M., Langedijk, A.C., van Tuinen, E., Geerse, R.H., Rauè, H.A., Maat, J., (1993) Effect of pmr1 disruption and different signal sequences on the intracellular processing and secretion of *Cyamopsis tetragonoloba* α-galactosidase by *S*. *cerevisiae*. Gene **125** 115-123
- Hollenberg, C. *et al*., EP-A-0096430 (GIST-BROCADES N.V., 1983))
- Hoogenboom H.R., Griffiths, A.D., Johnson, K.S., Chiswell, D.J., Hudson, P., and Winter, G. (1991) Multi-subunit proteins on the surface of filamentous phage: methodologies for displaying antibody (Fab) heavy and light chains. Nucleic Acids Research **15** 4133-4137
- Jones *et al*. (1986) Nature **321** 522
- Kriechbaum *et al*. (1989) FEBS Lett. **255** 63
- Ledeboer, A.M. *et al*., EP-A-0173378 (UNILEVER, 1986)
- Leenhouts, C.J. *et al*., EP-A-0487159 (UNILEVER, 1992)
- Lerner, Benkovic and Schultz, (1991) Science **252** 659-667
- Marks, J.D., Hoogenboom, H.R., Griffiths, A.D., and Winter, G. (1992) Molecular evolution of proteins on filamentous phage. J. Biol. Chem. **267** 16007-16010
- Meilhoc, E., Masson, J., Teissié, J. (1990) High efficiency transformation of intact yeast cells by electric pulses. Bio/Technology **8** 223-227
- Mitchell, P., (1979) Science **206** 1148-1159)
- Pessi et al. (1993) Nature **362** 367.
- Rouwenhorst, RJ., Visser, L.E., van der Baan, Scheffers, W.A., van Dijken, J.P. (1988) Production, distribution and kinetic properties of inulinase in continuous culture of *Kluyveromyces marxianus* CBS 6556. Appl. Environm. Microbiol. **54** 1131-1137.
- Sierkstra, L.N., Verbakel, J.M.A. and Verrips, C.T. (1991) Optimisation of a host/vector system for heterologous gene expression by *Hansenula polymorpha*. Current Genetics **19** 81-87.
- Skerra et al. (1988) Science **240** 1938
- Takahashi et al. (1993) Science **259** 1460-1463);
- Teeri et al., WO-A-93/02198 (TECH. RES. CENT. FINLAND, publ. 04.02.1993)
- Van Gorcom, R.F.M. *et al*., WO-A-91/19782 (UNILEVER, 1991)
- Wu et al. (1993) Bio/Technology **11** 71
- Zhou *et al*. (1991) Nucleic Acids Research **19** 6052

Additional references to prior-filed hut not prior-published patent applications, which are incorporated herein by reference:
- not prior-published PCT application EP 92/02896, filed 09.12.92 with priority date of 09.12.91 (UNILEVER / R.J. Gouka *et al*.), now publicly available as WO-A-93/12237
- not prior-published EP application 92202080.5, filed 08.07.92 (UNILEVER / F.M. Klis *et al*.), now publicly available as International (PCT) patent application WO-A-94/01567)
- not prior-published EP application 92402326.0, filed 21.08.92 (C. Casterman & R. Hamers), now publicly available as EP-A1-0 584 421
- not yet published EP application 92203932.6, filed 11.12.92 (UNILEVER / H.Y. Toschka & J.M.A. Verbakel).

Information on deposits of micro-organisms under the Budapest Treaty is given in Example 1 on page 23, lines 23-25 above. In agreement with Rule 28 (4) EPC, or a similar arrangement for a State not being a Contracting State of the EPC, it is hereby requested that a sample of such deposit, when requested, will be submitted to an expert only.

## Claims

1. A process for the production of:
(a) an antibody, or
(b) an antibody fragment, or
(c) a functionalised fragment, wherein one or more functional groups are attached resulting in a fusion product of such antibody fragment with another biofunctional molecule,
using a transformed lower eukaryotic host which is a mould or a yeast, wherein the yeast belongs to the genera Saccharomyces, Kluyveromyces, Hansenula, or Pichia,and which contains an expressible DNA sequence encoding the antibody or (functionalised) fragment thereof, which antibody or fragment or functionalised fragment is derived from a heavy chain immunoglobulin of Camelidae and is devoid of light chains.

2. A process according to claim 1, in which the mould belongs to the genera Aspergillus or Trichoderma.

3. A process according to claim 1,for the production of an antibody fragment or functionalised fragment which contains at least a variable domain of a heavy chain.

4. A process according to claim 1, in which the antibody or fragment or functionalised fragment derived from a heavy chain immunoglobulin of Camelidae comprises a complementarity determining region (CDR) different from the CDR belonging to the natural antibody ex Camelidae grafted on the framework of the variable domain of the heavy chain immunoglobulin ex Camelidae.

5. A process according to claim 1, wherein the heavy chain immunoglobulin is a catalytic immunoglobulin raised in Camelidae.

6. A process according to claim 1, in which the functionalised antibody or fragment thereof comprises a fusion protein of both a heavy chain immunoglobulin from Camelidae or a fragment thereof and another polypeptide.

7. A process according to claim 1, in which the DNA sequence encodes a modified heavy chain antibody or (functionalised) fragment thereof derived from Camelidae and being devoid of light chains, and is made by random or directed mutagenesis or both.

8. A process according to claim 7, in which the resulting antibody or (functionalised) fragment thereof is modified such that
- it is optimised for secretion by the lower eukaryotic host into the fermentation medium, or
- its binding properties (kₒₙ and k_{off}) are optimised, or
- it has acquired a metal chelating activity.

9. A transformed lower eukaryotic host which is a mould or a yeast, wherein the yeast belongs to the genera Saccharomyces, Kluyveromyces, Hansenula or Pichia, and which contains an expressible DNA sequence encoding an antibody or an antibody fragment, or functionalised fragment is derived from a heavy chain immunoglobulin of Camelidae and is devoid of light chains.

## Patentansprüche

1. Verfahren zur Herstellung von:
(a) einem Antikörper oder
(b) einem Antikörperfragment oder
(c) einem funktionalisierten Fragment, wobei eine oder mehrere funktionelle Gruppen gebunden sind, so dass ein Fusionsprodukt eines solchen Antikörperfragments mit einem anderen biofunktionellen Molekül resultiert,
unter Verwendung eines transformierten niederen eukaryotischen Wirts, der ein Schimmelpilz oder eine Hefe ist, wobei die Hefe zu den Genera Saccharomyces, Kluyveromy-ces, Hansenula oder Pichia gehört, und eine für den Antikörper oder das (funktionalisierte) Fragment davon kodierende exprimierbare DNA-Sequenz enthält, wobei der Antikörper oder das Fragment oder funktionalisierte Fragment von einem Schwere Kette Immunglobulin aus Camelidae abgeleitet und frei von Leichten Ketten ist.

2. Verfahren gemäß Anspruch 1, wobei der Schimmelpilz zu den Genera Aspergillus oder Trichoderma gehört.

3. Verfahren gemäß Anspruch 1 zur Herstellung eines Antikörperfragments oder funktionalisierten Fragments, das mindestens eine variable Domäne einer Schweren Kette enthält.

4. Verfahren gemäß Anspruch 1, wobei der von einem Schwere Kette Immunglobulin aus Camelidae abgeleitete Antikörper oder das Fragment oder das funktionalisierte Fragment eine Komplementaritat bestimmende Region (CDR) umfasst, die sich von der CDR unterscheidet, die zum natürlichen Antikörper aus Camelidae gehört, und auf das Gerüst der variablen Domäne des Schwere Kette Immunglobulins aus Camelidae aufgepfropft ist.

5. Verfahren gemäß Anspruch 1, wobei das Schwere Kette Immunglobulin ein in Camelidae erzeugtes katalytisches Immunglobulin ist.

6. Verfahren gemäß Anspruch 1, wobei der funktionalisierte Antikörper oder dessen Fragment ein Fusionsprotein von einem Schwere Kette Immunglobulin aus Camelidae oder einem Fragment davon und einem anderen Polypeptid umfasst.

7. Verfahren gemäß Anspruch 1, wobei die DNA-Sequenz einen modifizierten Schwere Kette Antikörper oder ein (funktionalisiertes) Fragment davon kodiert, der (das) aus Camelidae abgeleitet und frei von Leichten Ketten ist und durch zufällige oder gerichtete Mutagenese oder beides hergestellt ist.

8. Verfahren gemäß Anspruch 7, wobei der resultierende Antikörper oder das (funktionalisierte) Fragment davon so modifiziert ist, dass
- es für Sekretion durch den niederen eukaryotischen Wirt in das Fermentationsmedium optimiert ist oder
- seine Bindungseigenschaften (kₒₙ und k_{off}) optimiert sind oder
- es eine metallchelatisierende Aktivität angenommen hat.

9. Transformierter niederer eukaryotischer Wirt, der ein Schimmelpilz oder eine Hefe ist, wobei die Hefe zu den Genera Saccharomyces, Kluyveromyces, Hansenula oder Pichia gehört, und eine exprimierbare DNA-Sequenz enthält, die einen Antikörper oder ein Antikörperfragment oder ein funktionalisiertes Fragment kodiert, der (das) von einem Schwere Kette Immunglobulin von Camelidae abgeleitet ist und frei von Leichten Ketten ist.

## Revendications

1. Procédé pour la production
(a) d'un anticorps, ou
(b) d'un fragment d'anticorps, ou
(c) d'un fragment fonctionnalisé, dans lequel un ou plusieurs groupes fonctionnels sont attachés, pour donner un produit de fusion d'un tel fragment d'anticorps avec une autre molécule biofonctionnelle, à l'aide d'un hôte eucaryote inférieur transformé qui est une moisissure ou une levure, la levure appartenant aux genres Saccharomyces, *Kluyveromyces*, *Hansenula* ou *Pichia*, et qui contient une séquence d'ADN exprimable codant pour l'anticorps ou un fragment (fonctionnalisé) de celui-ci, lequel anticorps ou fragment ou fragment fonctionnalisé est issu d'une immunoglobuline à chaînes lourdes de camélidé et est dépourvu de chaînes légères.

2. Procédé selon la revendication 1, dans lequel la moisissure appartient au genre *Aspergillus* ou *Trichoderma*.

3. Procédé selon la revendication 1, pour la production d'un fragment ou fragment fonctionnalisé d'anticorps qui contient au moins un domaine variable d'une chaîne lourde.

4. Procédé selon la revendication 1, dans lequel l'anticorps ou fragment ou fragment fonctionnalisé issu d'une immunoglobuline à chaînes lourdes de camélidé comprend une région déterminant la complémentarité (CDR) différente de la CDR appartenant à l'anticorps naturel issu de camélidé greffé sur le squelette du domaine variable de l'immunoglobuline à chaînes lourdes issue de camélidé.

5. Procédé selon la revendication 1, dans lequel l'immunoglobuline à chaînes lourdes est une immunoglobuline catalytique suscitée chez des camélidés.

6. Procédé selon la revendication 1, dans lequel l'anticorps fonctionnalisé ou le fragment de celui-ci comprend une protéine de fusion ou à la fois une immunoglobuline à chaînes lourdes issue de camélidé ou un fragment de celle-ci et un autre polypeptide.

7. Procédé selon la revendication 1, dans lequel la séquence d'ADN code pour un anticorps à chaînes lourdes modifié ou un fragment (fonctionnalisé) de celui-ci, issu de camélidé et étant dépourvu de chaînes légères, et est produit par mutagenèse aléatoire ou dirigée ou les deux.

8. Procédé selon la revendication 7, dans lequel l'anticorps résultant ou le fragment (fonctionnalisé) de celui-ci est modifié de sorte que
- il est optimisé pour la sécrétion par l'hôte eucaryote inférieur dans le milieu de fermentation, ou
- ses propriétés de liaison (kₒₙ et k_{off}) sont optimisées, ou
- il a acquis une activité de chélation de métaux.

9. Hôte eucaryote inférieur transformé qui est une moisissure ou une levure, la levure appartenant aux genres *Saccharomyces*, *Kluyveromyces*, *Hansenula* ou *Pichia*, et qui contient une séquence d'ADN exprimable codant pour un anticorps ou un fragment (fonctionnalisé) de celui-ci, lequel anticorps ou fragment ou fragment fonctionnalisé est issu d'une immunoglobuline à chaînes lourdes de camélidé et est dépourvu de chaînes légères.


### SEQUENCE LISTING

(1) GENERAL INFORMATION:
   (i) APPLICANT:
      (A) NAME: Unilever N.V.
      (B) STREET: Weena 455
      (C) CITY: Rotterdam
      (E) COUNTRY: The Netherlands
      (F) POSTAL CODE (ZIP): NL-3013 AL

      (A) NAME: Unilever PLC
      (B) STREET: Unilever House Blackfriars
      (C) CITY: London
      (E) COUNTRY: United Kingdom
      (F) POSTAL CODE (ZIP): EC4P 4BQ

      (A) NAME: Leon Gerardus Joseph FRENKEN
      (B) STREET: Geldersestraat 90
      (C) CITY: Rotterdam
      (E) COUNTRY: The Netherlands
      (F) POSTAL CODE (ZIP): NL-3011 MP

      (A) NAME: Cornelis Theodorus VERRIPS
      (B) STREET: Hagedoorn 18
      (C) CITY: Maassluis
      (E) COUNTRY: The Netherlands
      (F) POSTAL CODE (ZIP): NL-3142 KB

      (A) NAME: Raymond HAMERS
      (B) STREET: Vijversweg 15
      (C) CITY: Sint-Genesius-Rode
      (E) COUNTRY: Belgium
      (F) POSTAL CODE (ZIP): B-1640

      (A) NAME: Cécile HAMERS-CASTERMAN
      (B) STREET: Vijversweg 15
      (C) CITY: Sint-Genesius-Rode
      (E) COUNTRY: Belgium
      (F) POSTAL CODE (ZIP): B-1640

      (A) NAME: Serge Victor Marie MUYLDERMANS
      (B) STREET: Brusselse Steenweg 55
      (C) CITY: Hoeilaart
      (E) COUNTRY: Belgium
      (F) POSTAL CODE (ZIP): B-1560
   (ii) TITLE OF INVENTION: Production of antibodies or (functionalized) fragments thereof derived from heavy chain immunoglobulins of Camelidae.
   (iii) NUMBER OF SEQUENCES: 62
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Ala Pro Glu Leu Leu
   1               5
(2) INFORMATION FOR SEQ ID NO: 2:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 2:
   Ala Pro Glu Leu Pro
   1               5
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 3:
   CGCCATCAAG GTACCAGTTG A                                              21
(2) INFORMATION FOR SEQ ID NO: 4:
   (i) SEQUENCE CHARACTERISTICS:
      (A) LENGTH: 89 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human heavy chain framework (subgroup III) (Xaa = CDR1, Xaa xaa = CDR2 and Xaa Xaa Xaa = CDR3)
   (xi) SEQUENCE DESCRIPTION: SEQ ID NO: 4:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Xaa Trp Val Arg Gln Ala
               20                  25                  30
   Pro Gly Lys Gly Leu Glu Trp Val Ser Xaa Xaa Arg Phe Thr Ile Ser
           35                  40                  45
   Arg Asp Asn Ser Lys Asn Thr Leu Tyr Leu Gln Met Asn Ser Leu Arg
       50                  55                  60
   Ala Glu Asp Thr Ala Val Tyr Tyr Cys Ala Arg Xaa Xaa Xaa Trp Gly
   65                  70                  75                  80
   Gln Gly Thr Leu Val Thr Val Ser Ser
                   85
(2) INFORMATION FOR SEQ ID NO: 5:
   (i) SEQUENCE CHARACTERISTICS:
      (A) LENGTH: 81 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" framework A (Xaa = CDR1, Xaa Xaa = CDR2 and Xaa Xaa Xaa = CDR3)
   (xi) SEQUENCE DESCRIPTION: SEQ ID NO: 5:
   Gly Gly Ser Val Gln Gly Gly Gly Ser Leu Arg Leu Ser Cys Ala Ile
   1               5                   10                  15
   Ser Gly Xaa Trp Phe Arg Glu Gly Pro Gly Lys Glu Arg Glu Gly Ile
               20                  25                  30
   Ala Xaa Xaa Arg Phe Thr Ile Ser Gln Asp Ser Thr Leu Lys Thr Met
           35                  40                  45
   Tyr Leu Leu Met Asn Asn Leu Lys Pro Glu Asp Thr Gly Thr Tyr Tyr
       50                  55                  60
   Cys Ala Ala Xaa Xaa Xaa Trp Gly Gln Gly Thr Gln Val Thr Val Ser
   65                  70                  75                  80
   Ser
(2) INFORMATION FOR SEQ ID NO: 6:
   (i) SEQUENCE CHARACTERISTICS:
      (A) LENGTH: 81 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" framework B
      (Xaa = CDR1, Xaa Xaa = CDR2 and Xaa Xaa Xaa = CDR3)
   (xi) SEQUENCE DESCRIPTION: SEQ ID NO: 6:
   Gly Gly Ser Val Gln Ala Gly Gly Ser Leu Arg Leu Ser Cys Ala Ser
   1               5                   10                  15
   Ser Ser Xaa Trp Tyr Arg Gln Ala Pro Gly Lys Glu Arg Glu Phe Val
               20                  25                  30
   Ser Xaa Xaa Arg Phe Thr Ile Ser Gln Asp Ser Ala Lys Asn Thr Val
           35                  40                  45
   Tyr Leu Gln Met Asn Ser Leu Lys Pro Glu Asp Thr Ala Met Tyr Tyr
       50                  55                  60
   Cys Lys Ile Xaa Xaa Xaa Trp Gly Gln Gly Thr Gln Val Thr Val Ser
   65                  70                  75                  80
   Ser
(2) INFORMATION FOR SEQ ID NO: 7:
   (i) SEQUENCE CHARACTERISTICS:
      (A) LENGTH: 37 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" framework - short hinge - CH2 fragment
   (xi) SEQUENCE DESCRIPTION: SEQ ID NO: 7:
   Trp Gly Gln Gly Thr Gln Val Thr Val Ser Ser Gly Thr Asn Glu Val
   1               5                   10                  15
   Cys Lys Cys Pro Lys Cys Pro Ala Pro Glu Leu Pro Gly Gly Pro Ser
               20                  25                  30
   Val Phe Val Phe Pro
           35
(2) INFORMATION FOR SEQ ID NO: 8:
   (i) SEQUENCE CHARACTERISTICS:
      (A) LENGTH: 60 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin"
      framework - long hinge - CH2 fragment
   (xi) SEQUENCE DESCRIPTION: SEQ ID NO: 8:
   Trp Gly Gln Gly Thr Gln Val Thr Val Ser Ser Glu Pro Lys Ile Pro
   1               5                   10                  15
   Gln Pro Gln Pro Lys Pro Gln Pro Gln Pro Gln Pro Gln Pro Lys Pro
               20                  25                  30
   Gln Pro Lys Pro Glu Pro Glu Cys Thr Cys Pro Lys Cys Pro Ala Pro
           35                  40                  45
   Glu Leu Leu Gly Gly Pro Ser Val Phe Ile Phe Pro
       50                  55                  60
(2) INFORMATION FOR SEQ ID NO: 9:
   (i) SEQUENCE CHARACTERISTICS:
      (A) LENGTH: 67 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human gamma-3 CH1 - hinge - CH2 fragment
   (xi) SEQUENCE DESCRIPTION: SEQ ID NO: 9:
   Lys Val Asp Lys Arg Val Glu Leu Lys Thr Pro Leu Gly Asp Thr Thr
   1               5                   10                  15
   His Thr Cys Pro Arg Cys Pro Glu Pro Lys Cys Ser Asp Thr Pro Pro
               20                  25                  30
   Pro Cys Pro Arg Cys Pro Glu Pro Lys Ser Cys Asp Thr Pro Pro Pro
           35                  40                  45
   Cys Pro Arg Cys Pro Ala Pro Glu Leu Leu Gly Gly Pro Ser Val Phe
       50                  55                  60
   Leu Phe Pro
   65
(2) INFORMATION FOR SEQ ID NO: 10:
   (i) SEQUENCE CHARACTERISTICS:
      (A) LENGTH: 35 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human gamma-1 CH1 - hinge - CH2 fragment
   (xi) SEQUENCE DESCRIPTION: SEQ ID NO: 10:
   Lys Val Asp Lys Lys Ala Glu Pro Lys Ser Cys Asp Lys Thr His Thr
   1               5                   10                  15
   Cys Pro Pro Cys Pro Ala Pro Glu Leu Leu Gly Gly Pro Ser Val Phe
               20                  25                  30
   Leu Phe Pro
           35
(2) INFORMATION FOR SEQ ID NO: 11:
   (i) SEQUENCE CHARACTERISTICS:
      (A) LENGTH: 31 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human gamma-2 CH1 - hinge - CH2 fragment
   (xi) SEQUENCE DESCRIPTION: SEQ ID NO: 11:
   Lys Val Lys Val Thr Val Glu Arg Lys Cys Cys Val Glu Cys Pro Pro
   1               5                   10                  15
   Cys Pro Ala Pro Pro Val Ala Gly Pro Ser Val Phe Leu Phe Pro
               20                  25                  30
(2) INFORMATION FOR SEQ ID NO: 12:
   (i) SEQUENCE CHARACTERISTICS:
      (A) LENGTH: 32 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human gamma-4 CH1 - hinge - CH2 fragment
   (xi) SEQUENCE DESCRIPTION: SEQ ID NO: 12:
   Lys Val Asp Lys Arg Val Glu Ser Lys Tyr Gly Pro Pro Cys Pro Ser
   1               5                   10                  15
   Cys Pro Ala Pro Glu Phe Leu Gly Gly Pro Ser Val Phe Leu Phe Pro
               20                  25                  30
(2) INFORMATION FOR SEQ ID NO: 13:
   (i) SEQUENCE CHARACTERISTICS:
      (A) LENGTH: 121 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: mouse heavy chain V-region
   (xi) SEQUENCE DESCRIPTION: SEQ ID NO: 13:
   Glu Val Lys Leu Val Glu Ser Gly Gly Gly Leu Val Gln Pro Gly Gly
   1               5                   10                  15
   Ser Leu Arg Leu Ser Cys Ala Thr Ser Gly Phe Thr Phe Ser Asp Phe
               20                  25                  30
   Tyr Met Glu Trp Val Arg Gln Pro Pro Gly Lys Arg Leu Glu Trp Ile
           35                  40                  45
   Ala Ala Ser Arg Asn Lys Ala Asn Asp Tyr Thr Thr Glu Tyr Ser Ala
       50                  55                  60
   Ser Val Lys Gly Arg Phe Ile Val Ser Arg Asp Thr Ser Gln Ser Ile
   65                  70                  75                  80
   Leu Tyr Leu Gln Met Asn Ala Leu Arg Ala Glu Asp Thr Ala Ile Tyr
                   85                  90                  95
   Tyr Cys Ala Arg Asp Tyr Tyr Gly Ser Ser Tyr Phe Asp Val Trp Gly
               100                 105                 110
   Ala Gly Thr Thr Val Thr Val Ser Ser
           115                 120
(2) INFORMATION FOR SEQ ID NO: 14:
   (i) SEQUENCE CHARACTERISTICS:
      (A) LENGTH: 131 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: human heavy chain V-region
   (xi) SEQUENCE DESCRIPTION: SEQ ID NO: 14:
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Xaa Ile Ser Xaa Lys Thr Asp Gly Gly Xaa Thr Tyr Tyr Ala Asp
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Xaa Xaa Xaa Xaa Xaa Xaa Xaa Xaa Xaa Xaa Xaa Tyr
               100                 105                 110
   Tyr Tyr Tyr His Xaa Phe Asp Tyr Trp Gly Gln Gly Thr Leu Val Thr
           115                 120                 125
   Val Ser Ser
       130
(2) INFORMATION FOR SEQ ID NO: 15:
   (i) SEQUENCE CHARACTERISTICS:
      (A) LENGTH: 114 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (1)
   (xi) SEQUENCE DESCRIPTION: SEQ ID NO: 15:
   Gly Gly Ser Val Gln Ala Gly Gly Ser Leu Arg Leu Ser Cys Ala Ala
   1               5                   10                  15
   Ser Gly Tyr Ser Asn Cys Pro Leu Thr Trp Ser Trp Tyr Arg Gln Phe
               20                  25                  30
   Pro Gly Thr Glu Arg Glu Phe Val Ser Ser Met Asp Pro Asp Gly Asn
           35                  40                  45
   Thr Lys Tyr Thr Tyr Ser Val Lys Gly Arg Phe Thr Met Ser Arg Gly
       50                  55                  60
   Ser Thr Glu Tyr Thr Val Phe Leu Gln Met Asp Asn Leu Lys Pro Glu
   65                  70                  75                  80
   Asp Thr Ala Met Tyr Tyr Cys Lys Thr Ala Leu Gln Pro Gly Gly Tyr
                   85                  90                  95
   Cys Gly Tyr Gly Xaa Cys Leu Trp Gly Gln Gly Thr Gln Val Thr Val
               100                 105                 110
   Ser Ser
(2) INFORMATION FOR SEQ ID NO: 16:
   (i) SEQUENCE CHARACTERISTICS:
      (A) LENGTH: 120 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (2)
   (xi) SEQUENCE DESCRIPTION: SEQ ID NO: 16:
   Asp Val Gln Leu Val Ala Ser Gly Gly Gly Ser Val Gln Ala Gly Gly
   1               5                   10                  15
   Ser Leu Arg Leu Ser Cys Thr Ala Ser Gly Asp Ser Phe Ser Arg Phe
               20                  25                  30
   Ala Met Ser Trp Phe Arg Gln Ala Pro Gly Lys Glu Cys Glu Leu Val
           35                  40                  45
   Ser Ser Ile Gln Ser Asn Gly Arg Thr Thr Glu Ala Asp Ser Val Gln
       50                  55                  60
   Gly Arg Phe Thr Ile Ser Arg Asp Asn Ser Arg Asn Thr Val Tyr Leu
   65                  70                  75                  80
   Gln Met Asn Ser Leu Lys Pro Glu Asp Thr Ala Val Tyr Tyr Cys Gly
                   85                  90                  95
   Ala Val Ser Leu Met Asp Arg Ile Ser Gln His Gly Cys Arg Gly Gln
               100                 105                 110
   Gly Thr Gln Val Thr Val Ser Leu
           115                 120
(2) INFORMATION FOR SEQ ID NO: 17:
   (i) SEQUENCE CHARACTERISTICS:
      (A) LENGTH: 123 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (3)
   (xi) SEQUENCE DESCRIPTION: SEQ ID NO: 17:
   Gly Gly Ser Val Gln Thr Gly Gly Ser Leu Arg Leu Ser Cys Ala Val
   1               5                   10                  15
   Ser Gly Phe Ser Phe Ser Thr Ser Cys Met Ala Trp Phe Arg Gln Ala
               20                  25                  30
   Ser Gly Lys Gln Arg Glu Gly Val Ala Ala Ile Asn Ser Gly Gly Gly
           35                  40                  45
   Arg Thr Tyr Tyr Asn Thr Tyr Val Ala Glu Ser Val Lys Gly Arg Phe
       50                  55                  60
   Ala Ile Ser Gln Asp Asn Ala Lys Thr Thr Val Tyr Leu Asp Met Asn
   65                  70                  75                  80
   Asn Leu Thr Pro Glu Asp Thr Ala Thr Tyr Tyr Cys Ala Ala Val Pro
                   85                  90                  95
   Ala His Leu Gly Pro Gly Ala Ile Leu Asp Leu Lys Lys Tyr Lys Tyr
               100                 105                 110
   Trp Gly Gln Gly Thr Gln Val Thr Val Ser Ser
           115                 120
(2) INFORMATION FOR SEQ ID NO: 18:
   (i) SEQUENCE CHARACTERISTICS:
      (A) LENGTH: 116 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (7)
   (xi) SEQUENCE DESCRIPTION: SEQ ID NO: 18:
   Gly Gly Ser Val Gln Gly Gly Gly Ser Leu Arg Leu Ser Cys Ala Ile
   1               5                   10                  15
   Ser Gly Tyr Thr Tyr Gly Ser Phe Cys Met Gly Trp Phe Arg Glu Gly
               20                  25                  30
   Pro Gly Lys Glu Arg Glu Gly Ile Ala Thr Ile Leu Asn Gly Gly Thr
           35                  40                  45
   Asn Thr Tyr Tyr Ala Asp Ser Val Lys Gly Arg Phe Thr Ile Ser Gln
       50                  55                  60
   Asp Ser Thr Leu Lys Thr Met Tyr Leu Leu Met Asn Asn Leu Lys Pro
   65                  70                  75                  80
   Glu Asp Thr Gly Thr Tyr Tyr Cys Ala Ala Glu Leu Ser Gly Gly Ser
                   85                  90                  95
   Cys Glu Leu Pro Leu Leu Phe Asp Tyr Trp Gly Gln Gly Thr Gln Val
               100                 105                 110
   Thr Val Ser Ser
           115
(2) INFORMATION FOR SEQ ID NO: 19:
   (i) SEQUENCE CHARACTERISTICS:
      (A) LENGTH: 114 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (9)
   (xi) SEQUENCE DESCRIPTION: SEQ ID NO: 19:
   Gly Gly Ser Val Gln Ala Gly Gly Ser Leu Thr Leu Ser Cys Val Tyr
   1               5                   10                  15
   Thr Asn Asp Thr Gly Thr Met Gly Trp Phe Arg Gln Ala Pro Gly Lys
               20                  25                  30
   Glu Cys Glu Arg Val Ala His Ile Thr Pro Asp Gly Met Thr Phe Ile
           35                  40                  45
   Asp Glu Pro Val Lys Gly Arg Phe Thr Ile Ser Arg Asp Asn Ala Gln
       50                  55                  60
   Lys Thr Leu Ser Leu Arg Met Asn Ser Leu Arg Pro Glu Asp Thr Ala
   65                  70                  75                  80
   Val Tyr Tyr Cys Ala Ala Asp Trp Lys Tyr Trp Thr Cys Gly Ala Gln
                   85                  90                  95
   Thr Gly Gly Tyr Phe Gly Gln Trp Gly Gln Gly Ala Gln Val Thr Val
               100                 105                 110
   Ser Ser
(2) INFORMATION FOR SEQ ID NO: 20:
   (i) SEQUENCE CHARACTERISTICS:
      (A) LENGTH: 125 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (11)
   (xi) SEQUENCE DESCRIPTION: SEQ ID NO: 20:
   Gly Gly Ser Val Gln Ala Gly Gly Ser Leu Arg Leu Ser Cys Asn Val
   1               5                   10                  15
   Ser Gly Ser Pro Ser Ser Thr Tyr Cys Leu Gly Trp Phe Arg Gln Ala
               20                  25                  30
   Pro Gly Arg Glu Arg Glu Gly Val Thr Ala Ile Asn Thr Asp Gly Ser
           35                  40                  45
   Ile Ile Tyr Ala Ala Asp Ser Val Lys Gly Arg Phe Thr Ile Ser Gln
       50                  55                  60
   Asp Thr Ala Lys Glu Thr Val His Leu Gln Met Asn Asn Leu Gln Pro
   65                  70                  75                  80
   Glu Asp Thr Ala Thr Tyr Tyr Cys Ala Ala Arg Leu Thr Glu Met Gly
                   85                  90                  95
   Ala Cys Asp Ala Arg Trp Ala Thr Leu Ala Thr Arg Thr Phe Ala Tyr
               100                 105                 110
   Asn Tyr Trp Gly Gln Gly Thr Gln Val Thr Val Ser Ser
           115                 120                 125
(2) INFORMATION FOR SEQ ID NO: 21:
   (i) SEQUENCE CHARACTERISTICS:
      (A) LENGTH: 114 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (13)
   (xi) SEQUENCE DESCRIPTION: SEQ ID NO: 21:
   Gly Gly Ser Val Glu Ala Gly Gly Ser Leu Arg Leu Ser Cys Thr Ala
   1               5                   10                  15
   Ser Gly Tyr Val Ser Ser Met Ala Trp Phe Arg Gln Val Pro Gly Gln
               20                  25                  30
   Glu Arg Glu Gly Val Ala Phe Val Gln Thr Ala Asp Asn Ser Ala Leu
           35                  40                  45
   Tyr Gly Asp Ser Val Lys Gly Arg Phe Thr Ile Ser His Asp Asn Ala
       50                  55                  60
   Lys Asn Thr Leu Tyr Leu Gln Met Arg Asn Leu Gln Pro Asp Asp Thr
   65                  70                  75                  80
   Gly Val Tyr Tyr Cys Ala Ala Gln Lys Lys Asp Arg Thr Arg Trp Ala
                   85                  90                  95
   Glu Pro Arg Glu Trp Asn Asn Trp Gly Gln Gly Thr Gln Val Thr Ala
               100                 105                 110
   Ser Ser
(2) INFORMATION FOR SEQ ID NO: 22:
   (i) SEQUENCE CHARACTERISTICS:
      (A) LENGTH: 122 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (16)
   (xi) SEQUENCE DESCRIPTION: SEQ ID NO: 22:
   Gly Gly Ser Ala Gln Ala Gly Gly Ser Leu Arg Leu Ser Cys Ala Ala
   1               5                   10                  15
   His Gly Ile Pro Leu Asn Gly Tyr Tyr Ile Ala Trp Phe Arg Gln Ala
               20                  25                  30
   Pro Gly Lys Gly Arg Glu Gly Val Ala Thr Ile Asn Gly Gly Arg Asp
           35                  40                  45
   Val Thr Tyr Tyr Ala Asp Ser Val Thr Gly Arg Phe Thr Ile Ser Arg
       50                  55                  60
   Asp Ser Pro Lys Asn Thr Val Tyr Leu Gln Met Asn Ser Leu Lys Pro
   65                  70                  75                  80
   Glu Asp Thr Ala Ile Tyr Phe Cys Ala Ala Gly Ser Arg Phe Ser Ser
                   85                  90                  95
   Pro Val Gly Ser Thr Ser Arg Leu Glu Ser Ser Asp Tyr Asn Tyr Trp
               100                 105                 110
   Gly Gln Gly Ile Gln Val Thr Ala Ser Ser
           115                 120
(2) INFORMATION FOR SEQ ID NO: 23:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (17)
   (xi) SEQUENCE DESCRIPTION: SEQ ID NO: 23:
   Gly Gly Ser Val Gln Pro Gly Gly Ser Leu Thr Leu Ser Cys Thr Val
   1               5                   10                  15
   Ser Gly Ala Thr Tyr Ser Asp Tyr Ser Ile Gly Trp Ile Arg Gln Ala
               20                  25                  30
   Pro Gly Lys Asp Arg Glu Val Val Ala Ala Ala Asn Thr Gly Ala Thr
           35                  40                  45
   Ser Lys Phe Tyr Val Asp Phe Val Lys Gly Arg Phe Thr Ile Ser Gln
       50                  55                  60
   Asp Asn Ala Lys Asn Thr Val Tyr Leu Gln Met Ser Phe Leu Lys Pro
   65                  70                  75                      80
   Glu Asp Thr Ala Ile Tyr Tyr Cys Ala Ala Ala Asp Pro Ser Ile Tyr
                   85                  90                  95
   Tyr Ser Ile Leu Xaa Ile Glu Tyr Lys Tyr Trp Gly Gln Gly Thr Gln
               100                 105                 110
   Val Thr Val Ser Ser
           115
(2) INFORMATION FOR SEQ ID NO: 24:
   (i) SEQUENCE CHARACTERISTICS:
      (A) LENGTH: 123 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (18)
   (xi) SEQUENCE DESCRIPTION: SEQ ID NO: 24:
   Gly Gly Ser Val Gln Ala Gly Gly Ser Leu Arg Leu Ser Cys Thr Gly
   1               5                   10                  15
   Ser Gly Phe Pro Tyr Ser Thr Phe Cys Leu Gly Trp Phe Arg Gln Ala
               20                  25                  30
   Pro Gly Lys Glu Arg Glu Gly Val Ala Gly Ile Asn Ser Ala Gly Gly
           35                  40                  45
   Asn Thr Tyr Tyr Ala Asp Ala Val Lys Gly Arg Phe Thr Ile Ser Gln
       50                  55                  60
   Gly Asn Ala Lys Asn Thr Val Phe Leu Gln Met Asp Asn Leu Lys Pro
   65                  70                  75                  80
   Glu Asp Thr Ala Ile Tyr Tyr Cys Ala Ala Asp Ser Pro Cys Tyr Met
                   85                  90                  95
   Pro Thr Met Pro Ala Pro Pro Ile Arg Asp Ser Phe Gly Trp Asp Asp
               100                 105                 110
   Phe Gly Gln Gly Thr Gln Val Thr Val Ser Ser
           115                 120
(2) INFORMATION FOR SEQ ID NO: 25:
   (i) SEQUENCE CHARACTERISTICS:
      (A) LENGTH: 119 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (19)
   (xi) SEQUENCE DESCRIPTION: SEQ ID NO: 25:
   Gly Gly Ser Val Gln Ala Gly Gly Ser Leu Arg Leu Ser Cys Ala Ala
   1               5                   10                  15
   Ser Asp Tyr Thr Ile Thr Asp Tyr Cys Met Ala Trp Phe Arg Gln Ala
               20                  25                  30
   Pro Gly Lys Glu Arg Glu Leu Val Ala Ala Ile Gln Val Val Arg Ser
           35                  40                  45
   Asp Thr Arg Leu Thr Asp Tyr Ala Asp Ser Val Lys Gly Arg Phe Thr
       50                  55                  60
   Ile Ser Gln Gly Asn Thr Lys Asn Thr Val Asn Leu Gln Met Asn Ser
   65                  70                  75                  80
   Leu Thr Pro Glu Asp Thr Ala Ile Tyr Ser Cys Ala Ala Thr Ser Ser
                   85                  90                  95
   Phe Tyr Trp Tyr Cys Thr Thr Ala Pro Tyr Asn Val Trp Gly Gln Gly
               100                 105                 110
   Thr Gln Val Thr Val Ser Ser
           115
(2) INFORMATION FOR SEQ ID NO: 26:
   (i) SEQUENCE CHARACTERISTICS:
      (A) LENGTH: 117 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (20)
   (xi) SEQUENCE DESCRIPTION: SEQ ID NO: 26:
   Gly Gly Ser Val Gln Val Gly Gly Ser Leu Arg Leu Ser Cys Val Ala
   1               5                   10                  15
   Ser Thr His Thr Asp Ser Ser Thr Cys Ile Gly Trp Phe Arg Gln Ala
               20                  25                  30
   Pro Gly Lys Glu Arg Glu Gly Val Ala Ser Ile Tyr Phe Gly Asp Gly
           35                  40                  45
   Gly Thr Asn Tyr Arg Asp Ser Val Lys Gly Arg Phe Thr Ile Ser Gln
       50                  55                  60
   Leu Asn Ala Gln Asn Thr Val Tyr Leu Gln Met Asn Ser Leu Lys Pro
   65                  70                  75                  80
   Glu Asp Ser Ala Met Tyr Tyr Cys Ala Ile Thr Glu Ile Glu Trp Tyr
                   85                  90                  95
   Gly Cys Asn Leu Arg Thr Thr Phe Thr Arg Trp Gly Gln Gly Thr Gln
               100                 105                 110
   Val Thr Val Ser Ser
           115
(2) INFORMATION FOR SEQ ID NO: 27:
   (i) SEQUENCE CHARACTERISTICS:
      (A) LENGTH: 125 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (21)
   (xi) SEQUENCE DESCRIPTION: SEQ ID NO: 27:
   Gly Gly Ser Val Gln Val Gly Gly Ser Leu Lys Leu Ser Cys Lys Ile
   1               5                   10                  15
   Ser Gly Gly Thr Pro Asp Arg Val Pro Lys Ser Leu Ala Trp Phe Arg
               20                  25                  30
   Gln Ala Pro Glu Lys Glu Arg Glu Gly Ile Ala Val Leu Ser Thr Lys
           35                  40                  45
   Asp Gly Lys Thr Phe Tyr Ala Asp Ser Val Lys Gly Arg Phe Thr Ile
       50                  55                  60
   Phe Leu Asp Asn Asp Lys Thr Thr Phe Ser Leu Gln Leu Asp Arg Leu
   65                  70                  75                  80
   Asn Pro Glu Asp Thr Ala Asp Tyr Tyr Cys Ala Ala Asn Gln Leu Ala
                   85                  90                  95
   Gly Gly Trp Tyr Leu Asp Pro Asn Tyr Trp Leu Ser Val Gly Ala Tyr
               100                 105                 110
   Ala Ile Trp Gly Gln Gly Thr His Val Thr Val Ser Ser
           115                 120                 125
(2) INFORMATION FOR SEQ ID NO: 28:
   (i) SEQUENCE CHARACTERISTICS:
      (A) LENGTH: 125 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (24)
   (xi) SEQUENCE DESCRIPTION: SEQ ID NO: 28:
   Gly Gly Ser Val Gln Ala Gly Gly Ser Leu Arg Leu Ser Cys Asn Val
   1               5                   10                  15
   Ser Gly Ser Pro Ser Ser Thr Tyr Cys Leu Gly Trp Phe Arg Gln Ala
               20                  25                  30
   Pro Gly Lys Glu Arg Glu Gly Val Thr Ala Ile Asn Thr Asp Gly Ser
           35                  40                  45
   Val Ile Tyr Ala Ala Asp Ser Val Lys Gly Arg Phe Thr Ile Ser Gln
       50                  55                  60
   Asp Thr Ala Lys Lys Thr Val Tyr Leu Gln Met Asn Asn Leu Gln Pro
   65                  70                  75                      80
   Glu Asp Thr Ala Thr Tyr Tyr Cys Ala Ala Arg Leu Thr Glu Met Gly
                   85                  90                  95
   Ala Cys Asp Ala Arg Trp Ala Thr Leu Ala Thr Arg Thr Phe Ala Tyr
               100                 105                 110
   Asn Tyr Trp Gly Arg Gly Thr Gln Val Thr Val Ser Ser
           115                 120                 125
(2) INFORMATION FOR SEQ ID NO: 29:
   (i) SEQUENCE CHARACTERISTICS:
      (A) LENGTH: 129 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (25)
   (xi) SEQUENCE DESCRIPTION: SEQ ID NO: 29:
   Gly Gly Ser Val Gln Thr Gly Gly Ser Leu Arg Leu Ser Cys Glu Ile
   1               5                   10                  15
   Ser Gly Leu Thr Phe Asp Asp Ser Asp Val Gly Trp Tyr Arg Gln Ala
               20                  25                  30
   Pro Gly Asp Glu Cys Lys Leu Val Ser Gly Ile Leu Ser Asp Gly Thr
           35                  40                  45
   Pro Tyr Thr Lys Ser Gly Asp Tyr Ala Glu Ser Val Arg Gly Arg Val
       50                  55                  60
   Thr Ile Ser Arg Asp Asn Ala Lys Asn Met Ile Tyr Leu Gln Met Asn
   65                  70                  75                  80
   Asp Leu Lys Pro Glu Asp Thr Ala Met Tyr Tyr Cys Ala Val Asp Gly
                   85                  90                  95
   Trp Thr Arg Lys Glu Gly Gly Ile Gly Leu Pro Trp Ser Val Gln Cys
               100                 105                 110
   Glu Asp Gly Tyr Asn Tyr Trp Gly Gln Gly Thr Gln Val Thr Val Ser
           115                 120                 125
   Ser
(2) INFORMATION FOR SEQ ID NO: 30:
   (i) SEQUENCE CHARACTERISTICS:
      (A) LENGTH: 111 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (27)
   (xi) SEQUENCE DESCRIPTION: SEQ ID NO: 30:
   Gly Gly Ser Val Gln Ala Gly Gly Ser Leu Arg Leu Ser Cys Ala Ser
   1               5                   10                  15
   Ser Ser Lys Tyr Met Pro Cys Thr Tyr Asp Met Thr Trp Tyr Arg Gln
               20                  25                  30
   Ala Pro Gly Lys Glu Arg Glu Phe Val Ser Ser Ile Asn Ile Asp Gly
   35 40 45
   Lys Thr Thr Tyr Ala Asp Ser Val Lys Gly Arg Phe Thr Ile Ser Gln
   50 55 60
   Asp Ser Ala Lys Asn Thr Val Tyr Leu Gln Met Asn Ser Leu Lys Pro
   65 70 75 80
   Glu Asp Thr Ala Met Tyr Tyr Cys Lys Ile Asp Ser Tyr Pro Cys His
   85 90 95
   Leu Leu Asp Val Trp Gly Gln Gly Thr Gln Val Thr Val Ser Ser
   100 105 110
(2) INFORMATION FOR SEQ ID NO: 31:
   (i) SEQUENCE CHARACTERISTICS:
      (A) LENGTH: 112 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: camel "heavy chain immunoglobulin" V-region (29)
   (xi) SEQUENCE DESCRIPTION: SEQ ID NO: 31:
   Gly Gly Ser Val Gln Ala Gly Gly Ser Leu Arg Leu Ser Cys Val Ala
   1 5 10 15
   Ser Gly Phe Asn Phe Glu Thr Ser Arg Met Ala Trp Tyr Arg Gln Thr
   20 25 30
   Pro Gly Asn Val Cys Glu Leu Val Ser Ser Ile Tyr Ser Asp Gly Lys
   35 40 45
   Thr Tyr Tyr Val Asp Arg Met Lys Gly Arg Phe Thr Ile Ser Arg Glu
   50 55 60
   Asn Ala Lys Asn Thr Leu Tyr Leu Gln Leu Ser Gly Leu Lys Pro Glu
   65 70 75 80
   Asp Thr Ala Met Tyr Tyr Cys Ala Pro Val Glu Tyr Pro Ile Ala Asp
   85 90 95
   Met Cys Ser Arg Tyr Gly Asp Pro Gly Thr Gln Val Thr Val Ser Ser
   100 105 110
(2) INFORMATION FOR SEQ ID NO: 32:
   (i) SEQUENCE CHARACTERISTICS:
      (A) LENGTH: 416 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: camel "heavy chain immunoglobulin" V-region followed by the FLAG sequence (pB03)
   (ix) FEATURE:
      (A) NAME/KEY: CDS
      (B) LOCATION: 1..408
   (xi) SEQUENCE DESCRIPTION: SEQ ID NO: 32:
   CAG GTG AAA CTG CTC GAG TCT GGG GGA GGC TCG GTG CAG GCT GGG GGG        48
   Gln Val Lys Leu Leu Glu Ser Gly Gly Gly Ser Val Gln Ala Gly Gly
     1               5                  10                  15
   TCT CTG ACA CTC TCT TGT GTA TAC ACC AAC GAT ACT GGG ACC ATG GGA        96
   Ser Leu Thr Leu Ser Cys Val Tyr Thr Asn Asp Thr Gly Thr Met Gly
                20                  25                  30
   TGG TTT CGC CAG GCT CCA GGG AAA GAG TGC GAA AGG GTC GCG CAT ATT       144
   Trp Phe Arg Gln Ala Pro Gly Lys Glu Cys Glu Arg Val Ala His Ile
            35                  40                  45
   ACG CCT GAT GGT ATG ACC TTC ATT GAT GAA CCC GTG AAG GGG CGA TTC       192
   Thr Pro Asp Gly Met Thr Phe Ile Asp Glu Pro Val Lys Gly Arg Phe
        50                  55                  60
   ACG ATC TCC CGA GAC AAC GCC CAG AAA ACG TTG TCT TTG CGA ATG AAT       240
   Thr Ile Ser Arg Asp Asn Ala Gln Lys Thr Leu Ser Leu Arg Met Asn
    65                  70                  75                  80
   AGT CTG AGG CCT GAG GAC ACG GCC GTG TAT TAC TGT GCG GCA GAT TGG       288
   Ser Leu Arg Pro Glu Asp Thr Ala Val Tyr Tyr Cys Ala Ala Asp Trp
                    85                  90                  95
   AAA TAC TGG ACT TGT GGT GCC CAG ACT GGA GGA TAC TTC GGA CAG TGG       336
   Lys Tyr Trp Thr Cys Gly Ala Gln Thr Gly Gly Tyr Phe Gly Gln Trp
               100                 105                 110
   GGT CAG GGG GCC CAG GTC ACC GTC TCC TCA CTA GCT AGT TAC CCG TAC       384
   Gly Gln Gly Ala Gln Val Thr Val Ser Ser Leu Ala Ser Tyr Pro Tyr
           115                 120                 125
   GAC GTT CCG GAC TAC GGT TCT TAATAGAATT C                              416
   Asp Val Pro Asp Tyr Gly Ser
       130                 135
(2) INFORMATION FOR SEQ ID NO: 33:
   (i) SEQUENCE CHARACTERISTICS:
      (A) LENGTH: 135 amino acids
      (B) TYPE: amino acid
      (D) TOPOLOGY: linear
   (ii) MOLECULE TYPE: protein
   (xi) SEQUENCE DESCRIPTION: SEQ ID NO: 33:
   Gln Val Lys Leu Leu Glu Ser Gly Gly Gly Ser Val Gln Ala Gly Gly
    1               5                  10                  15
   Ser Leu Thr Leu Ser Cys Val Tyr Thr Asn Asp Thr Gly Thr Met Gly
               20                  25                  30
   Trp Phe Arg Gln Ala Pro Gly Lys Glu Cys Glu Arg Val Ala His Ile
           35                  40                  45
   Thr Pro Asp Gly Met Thr Phe Ile Asp Glu Pro Val Lys Gly Arg Phe
       50                  55                  60
   Thr Ile Ser Arg Asp Asn Ala Gln Lys Thr Leu Ser Leu Arg Met Asn
   65                  70                  75                  80
   Ser Leu Arg Pro Glu Asp Thr Ala Val Tyr Tyr Cys Ala Ala Asp Trp
                   85                  90                  95
   Lys Tyr Trp Thr Cys Gly Ala Gln Thr Gly Gly Tyr Phe Gly Gln Trp
               100                 105                 110
   Gly Gln Gly Ala Gln Val Thr Val Ser Ser Leu Ala Ser Tyr Pro Tyr
           115                 120                 125
   Asp Val Pro Asp Tyr Gly Ser
       130                 135
(2) INFORMATION FOR SEQ ID NO: 34:
   (i) SEQUENCE CHARACTERISTICS:
      (A) LENGTH: 443 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: camel "heavy chain immunoglobulin" V-region followed by the FLAG sequence (pB09)
   (ix) FEATURE:
      (A) NAME/KEY: CDS
      (B) LOCATION: 1..435
   (xi) SEQUENCE DESCRIPTION: SEQ ID NO: 34:
(2) INFORMATION FOR SEQ ID NO: 35:
   (i) SEQUENCE CHARACTERISTICS:
      (A) LENGTH: 144 amino acids
      (B) TYPE: amino acid
      (D) TOPOLOGY: linear
   (ii) MOLECULE TYPE: protein
   (xi) SEQUENCE DESCRIPTION: SEQ ID NO: 35:
   Gln Val Lys Leu Leu Glu Ser Gly Gly Gly Ser Val Gln Thr Gly Gly
    1               5                  10                  15
   Ser Leu Arg Leu Ser Cys Ala Val Ser Gly Phe Ser Phe Ser Thr Ser
               20                  25                  30
   Cys Met Ala Trp Phe Arg Gln Ala Ser Gly Lys Gln Arg Glu Gly Val
           35                  40                  45
   Ala Ala Ile Asn Ser Gly Gly Gly Arg Thr Tyr Tyr Asn Thr Tyr Val
       50                  55                  60
   Ala Glu Ser Val Lys Gly Arg Phe Ala Ile Ser Gln Asp Asn Ala Lys
   65                  70                  75                  80
   Thr Thr Val Tyr Leu Asp Met Asn Asn Leu Thr Pro Glu Asp Thr Ala
                   85                  90                  95
   Thr Tyr Tyr Cys Ala Ala Val Pro Ala His Leu Gly Pro Gly Ala Ile
               100                 105                 110
   Leu Asp Leu Lys Lys Tyr Lys Tyr Trp Gly Gln Gly Thr Gln Val Thr
           115                 120                 125
   Val Ser Ser Leu Ala Ser Tyr Pro Tyr Asp Val Pro Asp Tyr Gly Ser
       130                 135                 140
(2) INFORMATION FOR SEQ ID NO: 36:
   (i) SEQUENCE CHARACTERISTICS:
      (A) LENGTH: 449 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: camel heavy chain immunoglobulin" V-region followed by the FLAG sequence (pB24)
   (ix) FEATURE:
      (A) NAME/KEY: CDS
      (B) LOCATION: 1..441
   (xi) SEQUENCE DESCRIPTION: SEQ ID NO: 36:
   CAG GTG AAA CTG CTC GAG TCT GGG GGA GGG TCG GTG CAG GCT GGA GGG      48
   Gln Val Lys Leu Leu Glu Ser Gly Gly Gly Ser Val Gln Ala Gly Gly
     1               5                  10                  15
   TCT CTG AGA CTC TCC TGT AAT GTC TCT GGC TCT CCC AGT AGT ACT TAT      96
   Ser Leu Arg Leu Ser Cys Asn Val Ser Gly Ser Pro Ser Ser Thr Tyr
                20                  25                  30
   TGC CTG GGC TGG TTC CGC CAG GCT CCA GGG AAG GAG CGT GAG GGG GTC     144
   Cys Leu Gly Trp Phe Arg Gln Ala Pro Gly Lys Glu Arg Glu Gly Val
            35                  40                  45
   ACA GCG ATT AAC ACT GAT GGC AGT GTC ATA TAC GCA GCC GAC TCC GTG     192
   Thr Ala Ile Asn Thr Asp Gly Ser Val Ile Tyr Ala Ala Asp Ser Val
        50                  55                  60
   AAG GGC CGA TTC ACC ATC TCC CAA GAC ACC GCC AAG AAA ACG GTA TAT     240
   Lys Gly Arg Phe Thr Ile Ser Gln Asp Thr Ala Lys Lys Thr Val Tyr
    65                  70                  75                  80
   CTC CAG ATG AAC AAC CTG CAA CCT GAG GAT ACG GCC ACC TAT TAC TGC     288
   Leu Gln Met Asn Asn Leu Gln Pro Glu Asp Thr Ala Thr Tyr Tyr Cys
                    85                  90                  95
   GCG GCA AGA CTG ACG GAG ATG GGG GCT TGT GAT GCG AGA TGG GCG ACC     336
   Ala Ala Arg Leu Thr Glu Met Gly Ala Cys Asp Ala Arg Trp Ala Thr
               100                 105                 110
   TTA GCG ACA AGG ACG TTT GCG TAT AAC TAC TGG GGC CGG GGG ACC CAG     384
   Leu Ala Thr Arg Thr Phe Ala Tyr Asn Tyr Trp Gly Arg Gly Thr Gln
           115                 120                 125
   GTC ACC GTC TCC TCA CTA GCT AGT TAC CCG TAC GAC GTT CCG GAC TAC     432
   Val Thr Val Ser Ser Leu Ala Ser Tyr Pro Tyr Asp Val Pro Asp Tyr
       130                 135                 140
   GGT TCT TAATAGAATT C                                                449
   Gly Ser
   145
(2) INFORMATION FOR SEQ ID NO: 37:
   (i) SEQUENCE CHARACTERISTICS:
      (A) LENGTH: 146 amino acids
      (B) TYPE: amino acid
      (D) TOPOLOGY: linear
   (ii) MOLECULE TYPE: protein
   (xi) SEQUENCE DESCRIPTION: SEQ ID NO: 37:
   Gln Val Lys Leu Leu Glu Ser Gly Gly Gly Ser Val Gln Ala Gly Gly
    1               5                  10                  15
   Ser Leu Arg Leu Ser Cys Asn Val Ser Gly Ser Pro Ser Ser Thr Tyr
               20                  25                  30
   Cys Leu Gly Trp Phe Arg Gln Ala Pro Gly Lys Glu Arg Glu Gly Val
           35                  40                  45
   Thr Ala Ile Asn Thr Asp Gly Ser Val Ile Tyr Ala Ala Asp Ser Val
       50                  55                  60
   Lys Gly Arg Phe Thr Ile Ser Gln Asp Thr Ala Lys Lys Thr Val Tyr
   65                  70                  75                  80
   Leu Gln Met Asn Asn Leu Gln Pro Glu Asp Thr Ala Thr Tyr Tyr Cys
                   85                  90                  95
   Ala Ala Arg Leu Thr Glu Met Gly Ala Cys Asp Ala Arg Trp Ala Thr
               100                 105                 110
   Leu Ala Thr Arg Thr Phe Ala Tyr Asn Tyr Trp Gly Arg Gly Thr Gln
           115                 120                 125
   Val Thr Val Ser Ser Leu Ala Ser Tyr Pro Tyr Asp Val Pro Asp Tyr
       130                 135                 140
   Gly Ser
   145
(2) INFORMATION FOR SEQ ID NO: 38:
   (i) SEQUENCE CHARACTERISTICS:
      (A) LENGTH: 119 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: See figure 6
   (xi) SEQUENCE DESCRIPTION: SEQ ID NO: 38:
   AATTTAGCGG CCGCCCAGGT GAAACTGCTC GAGTAAGTGA CTAAGGTCAC CGTCTCCTCA    60
   GAACAAAAAC TCATCTCAGA AGAGGATCTG AATTAATGAG AATTCATCAA ACGGTGATA    119
(2) INFORMATION FOR SEQ ID ND: 39:
   (i) SEQUENCE CHARACTERISTICS:
      (A) LENGTH: 120 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: See figure 6
   (xi) SEQUENCE DESCRIPTION: SEQ ID NO: 39:
(2) INFORMATION FOR SEQ ID NO: 40:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: See figure 6
   (xi) SEQUENCE DESCRIPTION: SEQ ID NO: 40:
   Ala Gln Val Lys Leu Leu Glu
   1               5
(2) INFORMATION FOR SEQ ID NO: 41:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: See figure 6
   (xi) SEQUENCE DESCRIPTION: SEQ ID NO: 41:
   Val Thr Val Ser Ser Glu Gln Lys Leu Ile Ser Glu Glu Asp Leu Asn
   1               5                   10                  15
(2) INFORMATION FOR SEQ ID NO: 42:
   (i) SEQUENCE CHARACTERISTICS:
      (A) LENGTH: 117 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: See figure 19
   (xi) SEQUENCE DESCRIPTION: SEQ ID NO: 42:
   AATTTAGTCG CGACAGGTGA AACTGCTCGA GTAAGTGACT AAGGTCACCG TCTCCTCAGA      60
   ACAAAAACTC ATCTCAGAAG AGGATCTGAA TTAATGAGAA TTCATCTTAA GGTGATA        117
(2) INFORMATION FOR SEQ ID NO: 43:
   (i) SEQUENCE CHARACTERISTICS:
      (A) LENGTH: 117 base pairs
      (B) TYPE: nucleic acid
      (C) STRANDEDNESS: single
      (D) TOPOLOGY: linear
   (ii) MOLECULE TYPE: DNA (genomic)
   (vii) IMMEDIATE SOURCE:
      (B) CLONE: See figure 19
   (xi) SEQUENCE DESCRIPTION: SEQ ID NO: 43:
   AGCTTATCAC CTTAAGATGA ATTCTCATTA ATTCAGATCC TCTTCTGAGA TGAGTTTTTG      60
   TTCTGAGGAG ACGGTGACCT TAGTCACTTA CTCGAGCAGT TTCACCTGTC GCGACTA        117
(2) INFORMATION FOR SEQ ID NO: 44:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: See figure 19
   (xi) SEQUENCE DESCRIPTION: SEQ ID NO: 44:
   Arg Gln Val Lys Leu Leu
   1 5
(2) INFORMATION FOR SEQ ID NO: 45:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: protein
   (vii) IMMEDIATE SOURCE:
      (B) CLONE: See figure 19
   (xi) SEQUENCE DESCRIPTION: SEQ ID NO: 45:
   Val Thr Val Ser Ser Glu Gln Lys Leu Ile Ser Glu Glu Asp Leu Asn
   1 5 10 15
(2) INFORMATION FOR SEQ ID NO: 46:
   (i) SEQUENCE CHARACTERISTICS:
      (A) LENGTH: 4 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 46:
   Gln Val Lys Leu
   1
(2) INFORMATION FOR SEQ ID NO: 47:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 47:
   Val Thr Val Ser Ser
   1               5
(2) INFORMATION FOR SEQ ID NO: 48:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 48:
   GTCACCGTCT CCTCATAATG A 21
(2) INFORMATION FOR SEQ ID NO: 49:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 49:
   AGCTTCATTA TGAGGAGACG 20
(2) INFORMATION FOR SEQ ID NO: 50:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 50:
   GTCACCGTCT CCTCATAATG ATCTTAAGGT GATA 34
(2) INFORMATION FOR SEQ ID NO: 51:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 51:
   AGCTTATCAC CTTAAGATCA TTATGAGGAG ACG 33
(2) INFORMATION FOR SEQ ID NO: 52:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 52:
   AATTGCGGCC GC 12
(2) INFORMATION FOR SEQ ID NO: 53:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 53:
   CATGCAGTCT TCGGGC 16
(2) INFORMATION FOR SEQ ID NO: 54:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 54:
   TTAAGCCCGA AGACTG 16
(2) INFORMATION FOR SEQ ID NO: 55:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 55:
   TCACTGAATT CGGGATCATG AGGACTCTCC TTGTGAGCTC GCTT 44
(2) INFORMATION FOR SEQ ID NO: 56:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 56:
   ATGTCACAAA GCTTAAGCAC GAAGACAGTC GACCGTGCGG CCGGAGAC 48
(2) INFORMATION FOR SEQ ID NO: 57:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 57:
   CGCGTCCATG CAGTCCTCAG GTGGATCATC CCAGGTGAAA CTGC 44
(2) INFORMATION FOR SEQ ID NO: 58:
   (i) SEQUENCE CHARACTERISTICS:
      (A) LENGTH: 44 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 58:
   TCGAGCAGTT TCACCTGGGA TGATCCACCT GAGGACTGCA TGGA 44
(2) INFORMATION FOR SEQ ID NO: 59:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 59:
   Ser Met Gln Ser Ser Gly Gly Ser Ser Gln Val Lys Leu Leu Glu
   1               5                   10                  15
(2) INFORMATION FOR SEQ ID NO: 60:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 60:
   CATGGCCAGG TGAAACTGCT CGAGTAAGTG ACTAAGGTCA CCGTCTCCTC AGC 53
(2) INFORMATION FOR SEQ ID NO: 61:
   (i) SEQUENCE CHARACTERISTICS:
      (A) LENGTH: 53 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 61:
   GGCCGCTGAG GAGACGGTGA CCTTAGTCAC TTACTCGAGC AGTTTCACCT GGC                53
(2) INFORMATION FOR SEQ ID NO: 62:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 62:
   Ser Ser Gly Gly Ser Ser
   1               5